# EUROPEAN PATENT APPLICATION

(11) **EP 0 601 804 A1**
(43) Date of publication of application: **15.06.1994**
(21) Application number: 93309706.5
(22) Date of filing: 03.12.1993
(51) Int. Cl.: A61L 27/00, A61L 31/00, A61L 33/00

(54) **Medical implants of biocompatible low modulus titanium alloy**

(30) Priority: 07.12.1992 US 986280; 24.03.1993 US 36414; 26.08.1993 US 112599
(71) Applicant: SMITH & NEPHEW RICHARDS, INC., Memphis, Tennessee 38116 (US)
(72) Inventor: Davidson, James A., Germantown, Tennessee 38139 (US); Kovacs, Paul, Memphis, Tennessee 38115 (US); Mishra, Ajit K., Memphis, Tennessee 38115 (US)
(74) Representative: Gilholm, Stephen Philip

(57) **Abstract**

Biocompatible medical implants from a high strength titanium alloy with low elastic modulus containing titanium, about 10-20 wt% or 35 to about 50 wt% niobium and up to 20 wt% zirconium. In particular, the titanium alloy has a modulus of elasticity closer to that of bone than other typically used metal alloys and does not include any elements which have been shown or suggested as having short or long term potential adverse effects from a standpoint of biocompatibility.

## Description

This invention relates to high strength, biocompatible metallic implants. In particular, the invention is of titanium alloy medical implants that have a low modulus of elasticity and high strength produced by a series of specific metallurgical steps to which the alloy is subjected. Further, the alloy does not include any elements which have been shown or suggested as having short term or long term potential adverse effects when implanted in the human body.

For many applications there has been, and there continues to be, a need for a metal that has a low modulus of elasticity but is also strong, fatigue-resistant, corrosion resistant, and has a hard surface that is resistant to abrasive wear. For instance, in the orthopaedic implant art, metals are still the most commonly used material for fabricating load-bearing implants such as, for instance, hip joints and knee joints.

Metals and metal alloys such as stainless steel, vitalium (cobalt alloy) and titanium have been used successfully. These materials have the requisite strength characteristics but typically have not been resilient or flexible enough to form an optimum implant material. Also, many alloys contain elements such as aluminium, vanadium, cobalt, nickel, molybdenum, and chromium which recent studies have suggested might have some long term adverse effects on human patients.

Many of the metal alloys typically used in prosthetic implants were developed for other applications, such as Ti-6A1-4V alloy in the aircraft industry. These alloys were later thought to be suitable for use as implant materials because they possess mechanical strength and appeared to have acceptable levels of biocompatibility. However, these metals typically have elastic moduli much higher than that of bone, for example, 316 stainless steel has an elastic modulus of about 200 GPa while that of cast heat-treated Co-Cr-Mo alloy is about 240 GPa.

It has also been found that many of these metals will corrode to some extent in body fluids thereby releasing ions that might possibly be harmful over a prolonged period of time. It is now believed that the corrosive effects of body fluids is due both to chemical and electro-chemical processes, with corrosion products forming when certain commonly-used metal alloys ionize from corrosion processes in the body. For example, aluminium metal ions have been associated with Alzheimer's disease and vanadium, cobalt, molybdenum, nickel and chromium are suspected of being toxic or carcinogenic.

It has been suggested that metals could be coated with a biocompatible plastic, ceramic or oxide to overcome the corrosion problem. However, coatings tend to wear off and are susceptible to delaminating and separating from the metal substrate, exposing the metal to body fluids.

Generally, it is the industry practice to passivate the implant metal alloys. However, passivation produces only thin, amorphous, poorly attached protective oxide films which have not proved totally effective in eliminating the formation of corrosion products in the body, particularly in situations where fretting occurs in the body.

As implant metals, titanium alloys offer advantages over stainless steels because of their lower susceptiblity to corrosion in the body coupled with their high strength and relatively low modulus of elasticity. Upon cooling, the currently used Ti-6A1-4V alloy transforms from a β-structure to an α plus β structure at about 1000°C. This transition can be shifted to a lower temperature by the addition of one or more suitable β-phase stabilizers such as molybdenum, zirconium, niobium, vanadium, tantalum, cobalt, chromium, iron, manganese and nickel.

Some efforts have been directed toward the development of alloys that eliminate harmful metals. For example, US patent 4,040,129 to Steinemann et al is directed to an alloy which includes titanium or zirconium as one component and, as a second component, any one or more of: nickel, tantalum, chromium, molybdenum or aluminum, but does not recognise or suggest any advantages from having a relatively low elastic modulus, or advantages or disadvantages associated with high temperature sintering treatments (at about 1250°C), commonly employed to attach porous metal coatings into which bone can grow to stabilize non-cemented, press-fit devices into the skeletal structure.

Although Steinemann provides that copper, cobalt, nickel, vanadium and tin should be excluded, apart from their presence as unavoidable impurities, the patent indicates that it is permissible to have any or all of chromium, molybdenum and aluminum, which are all believed to have potential long-term adverse effects, present in the alloy as long as their combined weight does not exceed 20% of the total weight of the alloy.

US patent 4,857,269 to Wang et al relates to a titanium alloy for a prosthetic implant said to have high strength and a low modulus. The titanium alloy contains up to 24 wt% of at least one isomorphous beta stabilizer from the group molybdenum, tantalum, zirconium and niobium; up to 3 wt% of at least one eutectoid beta stabilizer from the group iron, manganese, chromium, cobalt or nickel; and optionally up to 3 wt% of a metallic a-stabilizer from the group aluminium and lanthanum. Incidental impurities up to 0.05% carbon, 0.30% oxygen, 0.02% nitrogen, and up to 0.02% of the eutectoid former hydrogen are also included. Although there is some discussion of having an elastic modulus (eg. Young's modulus) around 85 GPa, the only examples of a low modulus (66.9-77.9 GPa) all contain 11.5 wt% Mo which is a potentially toxic element and undesirable for optimizing biocompatibility.

Other currently used metal alloys have similar drawbacks. For example, the commonly used Ti-6A1-4V alloy, with appropriate heat treatment, offers some degree of biocompatibility but has an elastic modulus of about 120 GPa. Although this elastic modulus is lower than other alloys and accordingly offers better load transfer to the surrounding bone, this modulus is still significantly greater than desired. Moreover, the alloy contains aluminium and also vanadium, which is now suspected to be a toxic or carcinogenic material when present in sufficient quantity.

Commercially available PROTOSUL 100 (Sulzer Bros. Ltd) is a Ti-6A1-7Nb alloy which intentionally avoids the potentially adverse effects of vanadium toxicity by substituting niobium. However, the alloy still contains aluminium and has an elastic modulus of about 110 GPa (15.9 x 10 psi) in heat-treated condition, and with a tensile strength of about 1060 MPa.

With medical prostheses being implanted in younger people and remaining in the human body for longer periods of time, there is a need for an implant material with requisite strength and flexibility requirements, which does not contain elements which are suspected as having long-term harmful effects on the human body. Desirably, the implant material should have a hardened surface or coating that is resistant to microfretting wear and gross mechanical wear.

Similarly, cardiovascular medical implants have unique blood biocompatibility requirements to ensure that the device is not rejected (as in the case of natural tissue materials for heart valves and grafts for heart transplants) or that adverse thrombogenic (clotting) or haemodynamic (blood flow) responses are avoided.

Cardiovascular implants, such as heart valves, can be fabricated from natural tissue. These bioprostheses can be affected by gradual calcification leading to the eventual stiffening and tearing of the implant.

Non-bioprosthetic implants are fabricated from materials such as pyrolytic carbon-coated graphite, pyrolytic carbon-coated titanium, stainless steel, cobalt-chrome alloys, cobalt-nickel alloys, alumina coated with polypropylene and poly-4-fluoroethylene.

For synthetic mechanical cardiovascular devices, properties such as the surface finish, flow characteristics, surface structure, charge, wear, and mechanical integrity all play a role in the ultimate success of the device. For example, typical materials used for balls and discs for heart valves include nylon, silicone, hollow titanium, **TEFLON** (Trade Mark), polyacetal, graphite, and pyrolytic carbon. Artificial hearts and ventricular assist devices are fabricated from various combinations of stainless steel, cobalt alloy, titanium, Ti-6A1-4V alloy, carbon fibre reinforced composites, polyurethanes, **BIOLON** (Trade Mark, DuPont), **HEMOTHANE** (Trade Mark, Sarns/3M), **DACRON** (Trade Mark), polysulfone, and other thermoplastics. Pacers, defibrillators, leads, and other similar cardiovascular implants are made of Ni-Co-Cr alloy, Co-Cr-Mo alloy, titanium and Ti-6A1-4V alloy, stainless steel, and various biocompatible polymers. Stents and vascular grafts are often made of **DACRON** (Trade Mark) stainless steel or other polymers. Catheters and guide wires are constructed from Co-Ni or stainless steel wire with surrounding polymer walls.

One of the most significant problems encountered in heart valves, artificial hearts, assist devices, pacers, leads, stents, and other cardiovascular implants is the formation of blood clots (thrombogenesis). Protein coatings are sometimes employed to reduce the risk of blood clot formation. Heparin is also used as an anti-thrombogenic coating.

It has been found that stagnant flow regions in the devices or non-optimal materials contribute to the formation of blood clots. These stagnant regions can be minimized by optimizing surface smoothness and minimizing abrupt changes in the size of the cross section through which the blood flows or minimizing either flow interference aspects. While materials selection for synthetic heart valves, and cardiovascular implants generally, is therefore dictated by a requirement for blood compatibility to avoid the formation of blood clots (thrombus), cardiovascular implants must also be designed to optimize blood flow and wear resistance.

Even beyond the limitations on materials imposed by the requirements of blood compatibility and limitations to designs imposed by the need to optimize blood flow, there is a need for durable designs since it is highly desirable to avoid the risk of a second surgical procedure to implant cardiovascular devices. Further, a catastrophic failure of an implanted device will almost certainly result in the death of the patient.

The most popular current heart valve designs include the St. Jude medical tilting disc double cusp (bi-leaf) valve. This valve includes a circular ring-like pyrolytic carbon valve housing or frame and a flow control element which includes pyrolytic carbon half-discs or leaves that pivot inside the housing to open and close the valve. The two leaves have a low profile and open to 85° from the horizontal axis.

Another popular heart valve is the Medtronic-Hall Valve wherein the flow control element is a single tilting disc made of carbon coated with pyrolytic carbon which pivots over a central strut inside a solid titanium ring-like housing. A third, less popular design, is the Omniscience valve which has a single pyrolytic disc as a flow control element inside a titanium housing. Finally, the Starr-Edwards ball and cage valves have a silastic ball riding inside a cobalt-chrome alloy cage. The cage is affixed to one side of a ring-like body for attachment to the heart tissue. More recent designs include trileaflet designs and concave bileaflet designs to improve blood flow.

From the point of view of durability, heart valves made of low-thrombogenic pyrolyte carbon could fail from disc or pivot joint wear or fracture related to uneven pyrolytic carbon coating, fracture of the ball cage, disc impingement, strut wear, disc wear, hinge failure, and weld failure. A more recent heart valve, the Baruah Bileaflet is similar to the St. Jude design but opens to 80° and is made of zirconium metal. The valve has worked well over its approximately two-year history with roughly 200 implants to date in India. This performance can be partly attributed to the lower elastic modulus of zirconium (about 90 GPa) and the resultant lower contact stress severity factor (Cc of about 0.28 x 10⁻⁷m) when the disc contacts the frame. In contrast, pyrolytic constructions produce contact stress severity factors of about 0.54 x 10⁻⁷m.

Although zirconium has worked well to date and can reduce contact stress severity, zirconium metal is relatively soft and sensitive to fretting wear. This is partly due to hard, loosely attached, naturally-present passive oxide surface films (several nanometers in thickness) which can initiate microabrasion and wear of the softer underlying metal. However, this naturally present zirconium oxide passive film is thrombogenically compatible with blood and the design is acceptable from a haemodynamic standpoint. Therefore, while the zirconium bileaflet valve appears to meet at least two of the major requirements for cardiac valve implants, namely blood compatibility and design for minimum stagnant flow regions, the use of soft zirconium metal leads to a relatively high rate of fretting wear and leads to the expectation that the valve may be less durable than one produced from materials less susceptible to fretting wear. Titanium and titanium alloys present a similar limitation, and Co-Cr-Mo, stainless steel, and Co-Ni alloys have much greater elastic modulus.

There exists a need for a metallic cardiac valve implant that is biocompatible, compatible with blood in that it does not induce blood clotting and does not form a calcified scale, that is designed to minimize stagnant flow areas where blood clotting can be initiated, that has a low elastic modulus for lower contact stress severity factors to ensure resistance to wear from impact, and that has a surface that is also resistant to microabrasion thereby enhancing durability.

Heart diseases, many of which cannot be cured by conventional surgery or drug therapy, continue to be a leading cause of death. For the seriously ill patient, heart replacement is often one of the few viable options available.

Recently, research and development has been carried out into permanently implantable, electrically driven, total artificial hearts (TAHs). The pumping mechanism of the TAHs would be implanted into the chest cavity of the patient and the device would be powered by a battery pack and a small transformer, worn by the patient, which transmits energy to the heart with no physical connections through the skin.

The development of TAHs posed several issues. Firstly, it was necessary to duplicate the action of a human heart, ensure long-term reliability and biocompatibility, while producing a device that fits into the chest cavity in terms of both its total volume and the orientation of its connections to natural vessels in the body. Aside from the purely mechanical, wear, and power supply issues, it is also necessary that the design and materials prevent infection and thrombosis. Blood is a non-Newtonian fluid and its properties, such as viscosity, change with oxygen content, kidney infection, and even the age of the patient. Further, plasma contains a suspension of fragile red blood cells which may be caught in artificial valves, or other mechanically stressful areas, thereby destroying these cells. It is therefore necessary to develop a TAH that does not stress blood components, and to fabricate the pump from materials that are not only biocompatible, but also "blood compatible" in the sense of minimizing damage to blood components and minimizing the formation of blood clots.

Many of the above comments also apply to ventricular assist devices (VADs), one of which is being developed by the Novacor Division of Baxter Health Care Corp. In the use of a VAD, the patient's heart remains in place while the VAD boosts the pumping pressure of the left ventricle of the heart. Consequently, the VAD is an assist device rather than a replacement. However, the VAD must be blood compatible for the same reasons as the total artifical heart.

There exists a need for a material that is lightweight, readily formable into complex shapes, biocompatible, and blood and tissue compatible with a hard surface that is resistant to abrasive wear, microfretting wear, and the corrosive effects of body fluids, for use in heart assist or replacement devices (including EMHs, VADs, and TAHs) to prolong the life of mechanical components while at the same time minimizing any deterious effect on blood components.

According to the invention we provide a biocompatible medical implant of low modulus and high strength for implantation into a living body where it is subject to corrosive effects of body fluids, said medical implant comprising:
a metallic alloy consisting essentially of:
(i) titanium;
(ii) from about 10 to 20 wt% niobium or from about 35 to about 50 wt% niobium; and
(iii) optionally up to about 20 wt% zirconium;

wherein said medical implant has an elastic modulus less than about 90 GPa and the corrosive effects of body fluids does not result in release of toxic or potentially toxic ions into the living body after surgical implantation of the implant into said body.

The invention provides novel medical implants fabricated from hot worked, high strength, low modulus alloys of titanium, niobium and zirconium. The alloys are preferably free of toxic or potentially toxic compositions when used as an implant fabrication alloy. More specifically, the invention alloy comprises titanium and niobium and optionally zirconium. To achieve the lowest modulus, the titanium should preferably be alloyed with from about 10 wt% to about 20 wt% niobium or from about 35 wt% to about 50 wt% niobium. Zirconium is an optional component preferably present in an amount from about 0 to about 20 wt%. Most preferably the invention alloy comprises about 74 wt% titanium, about 13 wt% zirconium and about 13 wt% niobium.

By the term medical implant we mean, eg. knee joint, consisting of a femoral component and a tibial base component; but particularly cardiovascular implants, that is devices for implanting and use within the cardiovascular system; and implants for use in conjunction with orthopaedic implants, eg. bone plates, bone screws, intramedullary rods and compression hip screws.

The invention also provides cardiovascular implants of a low modulus, biocompatible, hemocompatible, metallic alloy of titanium with niobium and optionally zirconium including heart valves, artificial hearts, ventricular assist devices, defibrillators, pacers, electrical leads, sensors, grafts, stents, and catheter devices. The invention also provides surface hardened versions of these devices produced by oxygen or nitrogen diffusion hardening to improve resistance to cavitation, microfretting wear, and impact-induced wear.

The inherently low modulus of Ti-Nb-Zr alloys, between about 6 to about 12 million psi depending on metallurgical treatment and composition, provide a more flexible and forgiving construct for cardiovascular applications while improving contact stress levels, valve closure, and the ability of leaves in certain valve designs to self-align with blood flow and reduce thrombodynamic effects.

The invention provides components for use in mechanical heart replacement or assist devices, such as external mechanical hearts (EMHs), total artificial hearts (TAHs), and ventricular assist devices (VADs), that are lightweight, while also being resistant to corrosive body fluids, mechanical wear, abrasive wear, and microfretting wear. Further, the components of reduced risk of thrombogenesis (blood clotting).

The preferred low modulus titanium alloys of the invention for use as cardiovascular implants have the compositions: (i) titanium; about 10 wt% to about 20 wt% niobium; and optionally from about 0 wt% to about 20 wt% zirconium; and (ii) titanium; about 35 wt% to about 50 wt% niobium; and optionally from about 0 wt% to about 20 wt% zirconium. Tantalum can also be present as a substitute for Nb. These alloys are referred to herein as "Ti-Nb-Zr alloys", even though tantalum may also be present.

The exclusion of elements besides titanium, zirconium, and niobium, or tantalum results in an alloy which does not contain known toxins or carcinogens, or elements that are known or suspected of including diseases or adverse tissue response in the long term.

Without the presence of zirconium in the composition, the ability of the Ti-Nb-Zr alloy to surface harden during oxygen or nitrogen diffusion hardening treatments is more limited. Therefore, presence of zirconium is especially preferred when the alloy implant must be diffusion hardened. Other non-toxic filler materials such as tantalum, which stabilize the β-phase of titanium alloy, but do not affect the low modulus, (the modulus of elasticity), ie. maintain it at less than about 85 GPa, could also be added. The alloy may be substantially in the β-phase and have a strength of greater than 620 MPa.

A porous coating, such as plasma-sprayed or sintered titanium or titanium alloy (including Ti-Nb-Zr alloy) beads or wire mesh may also be added to the implant's surfaces to improve tissue attachment, such as the formation of an endothelial cell layer, preferred in artificial heart, ventricular assist devices, grafts, and stent devices. Such coatings provide more favourable blood interaction and flow characteristics, and also tend to stabilize the implant with the body. Thus, such porous coatings may also be useful for connecting regions of these devices as well as for heart valves and grafts. Even though the application of such porous coatings usually requires sintering at relatively high temperatures, the properties of the Ti-Nb-Zr alloy that might affect its usefulness as an implant material are not adversely affected.

The invention alloy is strengthened by a hot working process wherein the alloy is heated to a temperature about its β-transus, or within about 100°C below its β-transus, hot worked, and then cooled rapidly, following which it is aged at temperatures below the β-transus. Preferably, this aging is carried out for about 2 to about 8 hours, most preferably about 6 hours at about 500°C. The aging process may also consist of a gradual ramp-up from room temperature, preferably in about 0.5 to 10 hours, during which preaging of the material may occur, followed by isothermal aging at an appropriate temperature below the β-transus for from about 15 minutes to 20 hours, preferably about 6 hours. Note that by the time the alloy is removed from the furnace and the hot working operation performed, the temperature of the alloy may have decreased significantly; so the hot working operation may actually occur at a temperature significantly lower than the temperature to which the alloy is heated prior to hot working. The invention's hot worked, quenched and aged titanium alloys have a low elastic modulus (about 60 to about 90 GPa) and have tensile strengths exceeding about 700 MPa, preferably exceeding about 800 MPa.

Further, the invention implants may be surface hardened by any one of several processes used in the field of metallurgy but not necessarily known for use with medical implants. For example, there are processes in which the alloy is subjected to nitrogen or oxygen diffusion, internal oxidation, or nitrogen or oxygen ion implantation. A description of these techniques may be found in our copending application, US Serial No.832,735, filed 7 February 1992, which is hereby incorporated by reference as if fully set forth. Clearly, the alloy should be formed into the desired shape for its intended use before surface hardening or the benefits of surface hardness may be lost in any subsequent shaping operations that affect or remove the surface of the alloy to a significant extent. Further, the shaped alloy may be worked for strength enhancement before surface hardening.

The most preferred hot worked, low modulus, high strength alloy for making medical implants contains about 74 wt% titanium, and about 13 wt% each of zirconium and niobium. Other elements are not deliberately added, but may be present in trace amounts to the extent that they were present as unavoidable impurities in the metals used to produce the alloy. Other non-toxic filler materials such as tantalum, which could be used to stabilize the β-phase, but not affect the low modulus (ie. maintain it less than about 90GPa), could also be added. The exclusion of elements besides titanium, zirconium and niobium or tantalum results in an alloy which does not contain known toxins or carcinogens or elements that are known or suspected of including diseases or adverse tissue response in the long term. Such an alloy is particularly useful in medical implant applications.

The medical implant according to the invention may comprise at least a partial outer surface protective coating selected from the group consisting of the oxides, nitrides, carbides and carbonitrides of elements of the metal alloy. It may further comprise a protective coating of amorphous diamond-like carbon on at least a portion of an outer surface of the implant.

The metallic alloy may be internally oxidised or nitrided beneath outer surfaces of the implant to produce a hardened medical implant.

We also provide a heart valve prosthesis for implantation in living body tissue of a patient, the heart valve having enhanced hemocompatibility, comprising:
(a) a valve body having an aperture through which blood is able to flow when the heart valve is implanted in a patient, the valve body fabricated from a metal alloy comprising:
   (i) titanium;
   (ii) from about 10 to about 20 wt% niobium or from about 35 to about 50 wt% niobium; and
   (iii) optionally up to about 20 wt% zirconium;
(b) a flow control element able to move relative to the valve body to close the aperture in the valve body thereby blocking blood flow through the aperture; and
(c) means, attached to the valve body, for restraining said flow control component to close proximity to the aperture in the valve body.

We also provide a ventricular assist device including components with surfaces in contact with blood when the device is implanted in a patient, the improvement comprising:
said components fabricated from a metal alloy comprising:
(i) titanium;
(ii) from about 10 to about 20 wt% niobium or from about 35 to about 50 wt% niobium; and
(iii) optionally up to about 20 wt% zirconium.

A total artificial heart device is provided for implantation into a chest cavity of a patient, the device including components with surfaces subject to mechanical wear and microfretting wear when in use in the patient, the improvement comprising:
(i) titanium;
(ii) from about 10 to about 20 wt% niobium or from about 35 to about 50 wt% niobium; and
(iii) optionally up to about 20 wt% zirconium.

In a total artificial heart device for implantation into a chest cavity of a patient, the device including components presenting surfaces that are in contact with blood when in use in the patient, the improvement wherein the components are fabricated from an alloy comprising:
(i) titanium;
(ii) from about 10 to about 20 wt% niobium or from about 35 to about 50 wt% niobium; and
(iii) optionally up to about 20 wt% zirconium.

We also provide a flexible guide wire for insertion into a living body to perform surgical operations, the guide wire comprising:
(a) an elongate, flexible guide wire body having a distal end, for insertion into a patient and into a catheter, and a proximal end for controlling the guide wire;
(b) an elongate guide wire core disposed internally along the longitudinal axis of the elongate body, said core comprising a metal alloy comprising:
   (i) titanium;
   (ii) from about 10 to about 20 wt% niobium or from about 35 to about 50 wt% niobium; and
   (iii) optionally up to about 20 wt% zirconium.

There is also provided an expandable stent for supporting a blood, urinary, or gastrointestinal vessel from collapsing inward, the stent comprising:
(a) a radially outwardly expandable substantially cylindrical stent body of a metal alloy comprising:
(i) titanium;
(ii) from about 10 to about 20 wt% niobium or from about 35 to about 50 wt% niobium; and
(iii) optionally up to about 20 wt% zirconium.

the stent body in its unexpected state sized for ease of insertion into a vessel and in expanded state sized for propping open a vessel, the stent body having a bore therethrough for receiving a means for expanding the body radially outwardly.

A biocompatible lead or sensor for conducting electrical signals to or from an organ in a living body, the lead comprising:
an elongate flexible body having distal and proximal ends, the flexible body comprising:
(a) an electrically conductive core of a metal alloy comprising:
(i) titanium;
(ii) from about 10 to about 20 wt% niobium or from about 35 to about 50 wt% niobium; and
(iii) optionally up to about 20 wt% zirconium.

A low modulus, biocompatible, percutaneous implant is provided that penetrates the skin of a living body and thereby protrudes from the body, the implant comprising:
(a) a low modulus metallic implant body fabricated from a metal alloy comprising:
(i) titanium;
ii) from about 10 to about 20 wt% niobium or from about 35 to about 50 wt% niobium; and
(iii) optionally up to about 20 wt% zirconium.

said implant body having a first end for insertion into said patient and a portion with a second end for extending outside of said patient.

There is provided an external mechanical heart including components with surfaces subject to mechanical wear and microfretting wear, the improvement comprising:
components of a metal alloy comprising:
(a) titanium;
(b) from about 10 to about 20 wt% niobium or from about 35 to about 50 wt% niobium; and
(c) optionally up to about 20 wt% zirconium.

In an external mechanical heart including components that contact blood when said heart is used to supply blood to a patient, the improvement comprising the components fabricated from a metal alloy comprising:
(a) titanium;
(b) from about 10 to about 20 wt% niobium or from about 35 to about 50 wt% niobium; and
(c) optionally up to about 20 wt% zirconium.

A vascular graft of enhanced durability, crush-resistance, low thrombogenicity, and hemocompatibility, said graft comprising:
(a) titanium;
(b) from about 10 to about 20 wt% niobium or from about 35 to about 50 wt% niobium; and
(c) optionally up to about 20 wt% zirconium.

said tubular body having a bore therethrough, said tubular body sized to replace a section of removed blood vessel.

The cardiovascular devices may comprise a metal alloy comprising from about 0.5 to about 20 wt% zirconium. The outer surfaces of the devices may be hardened by a process selected from the group consisting of oxygen diffusion hardening, nitrogen hardening, physical vapour deposition, and chemical vapour deposition. The devices may further comprise a coating overlayed over outer surfaces comprising a medicament. In addition it may comprise wear-resistant surfaces produced by a process selected from the group consisting of boronation and silver doping.

The invention's hot worked, low modulus, high strength titanium implants are produced by heating to above the β-transus temperature (or within the range including those temperatures below and within less than about 100°C of the β-transus temperature); hot working the implant; rapidly cooling the hot worked alloy to about room temperature; then reheating and aging at temperatures below the β-transus, in the range of 350-550°C, preferably about 500°C, for a time sufficient to provide an implant of adequate strength.

In a particularly preferred aging process, the implant is preaged by gradually heating the quenched implant for a period of time up to about 350-550°C. Thereafter, the preaged implant is isothermally aged at this temperature for a time sufficient to develop strength and hardness characteristics required.

The invention implants have a low modulus of elasticity of less than about 90 GPa. This is a significant improvement over Ti-6A1-4V which has a modulus of elasticity of about 120 GPa.

In certain applications it may still be desirable to coat the implant's surface with wear-resistant coatings such as amorphous diamond-like carbon coatings, zirconium dioxide coatings, titanium nitrides, carbides, or the like for protection against potential micro-fretting wear such as might occur on the bearing surfaces of implant prostheses.

A porous coating, such as a bead, powder, or wire mesh coating may be applied to implants of many types for a variety of applications fabricated from the inventive alloy. Such coatings are often useful to provide interstitial spaces for bone or tissue ingrowth into the implant, which tends to stabilize the implant in the skeletal structure.

While implants fabricated from the invention hot worked alloy possess high strength, the usefulness of these prostheses is not limited to load-bearing applications. Because of its corrosion resistance non-toxicity and relatively low modulus of elasticity, the alloy can be used to fabricate many types of medical implants including, but not limited to, knee joints, cheek bones, tooth implants, skull plates, fracture plates, intramedullary rods, staples, bone screws, spinal implants, pelvic plates, and other implants, cardiovascular implants such as synthetic heart valves, ventricular assist devices, total artificial hearts, stents, grafts, pacers, pacemaker leads and other electrical leads and sensors, defibrillators, guide wires and catheters, and percutaneous devices.

Figure 1 shows a simplified representation of a ball valve, like the Starr-Edwards Valve.

Figure 2 is a simplified representation of a disc valve.

Figure 3 is a simplified representation of a tilting disc, single cusp valve like the Medtronic-Hall valve.

Figure 4 is a simplified representation of a tilting disc, double cusp or bileaflet valve of the St. Jude or Baruah type.

Figure 5 is a schematic diagram of the Penn State/Sarns/3M design total artificial heart.

Figure 6 is a schematic diagram of the University of Utah total artificial heart.

Figure 7 is a schematic diagram of the Cleveland Clinic/Nimbus Inc total artificial heart.

Figure 8A is a schematic diagram, in cross section, of the Texas Heart Institute/Abiomed total artifical heart.

Figure 8B is a side view, in cross section, of the Texas Heart Institute/Abiomed total artifical heart of Figure 8A.

Figures 9A and B are schematic diagrams of end and front views, respectively, of a ventricular assist device.

Figure 10 is a schematic diagram of a vascular graft of woven metallic wire composition.

Figure 11A is a schematic diagram showing a balloon expandable stent positioned within a segment of a blood vessel to be propped open.

Figure 11B is a schematic diagram showing a balloon expanding stent into position within a blood vessel.

Figure 11C is a schematic diagram of a stent expanded in a blood vessel.

Figure 12A is a schematic diagram of the components of a defibrillator, showing power source, lead wire, and polymeric patch with coiled electrode.

Figure 12B is a cross section of the lead wire of Figure 12A.

Figure 13A is schematic diagram, in partial cross section, of the distal end of a guide wire.

Figure 13B is a cross section of the guide wire with coating thickness exaggerated.

Figure 13C shows a catheter containing coiled wire and polymer wall.

Figures 14A-C are schematic diagrams of prior art pacemaker leads with polyurethane covering.

Figure 14D is a schematic of an embodiment of the invention Ti-Nb-Zr pacemaker leads.

Figure 15 is a schematic diagram of a modular knee joint prosthesis.

Figure 16A is a schematic diagram of a side view of a typical intramedullary rod used in orthopaedic applications.

Figure 16B is a view of 16A, rotated by 90°.

Figure 17A is a schematic diagram of a typical bone screw.

Figure 17B is an end view of 17A.

Figure 18 is a schematic diagram showing a typical screw for fixing bone plates.

Figure 19 is a schematic diagram of a compression hip screw.

Figure 20A is a side view of a typical bone plate, in partial cross-section, for securing to the hip.

Figure 20B is the plate of 20A rotated by 90° to show 6 holes for bone screws to affix the plate in the body and a topmost hole for receiving a compression hip screw, like that of Figure 19.

Figure 20C is a cross-sectional view of 20B taken at 9C-9C.

The inventive alloy implants may be produced by combining, as commercially pure components, titanium, niobium and optionally zirconium in the appropriate proportions, heating the alloy to above its β-transus (or within the range of temperatures below and within about 100°C of the β-transus), hot working, rapidly cooling to about room temperature, and aging the alloy at temperatures below the β-transus for a sufficient length of time to allow strength development. It is essential that cooling be carried out rapidly, as by quenching with water. Conventional convective air cooling is not sufficiently rapid to produce the high strength, low modulus alloy of the invention after aging.

### Heart Valves

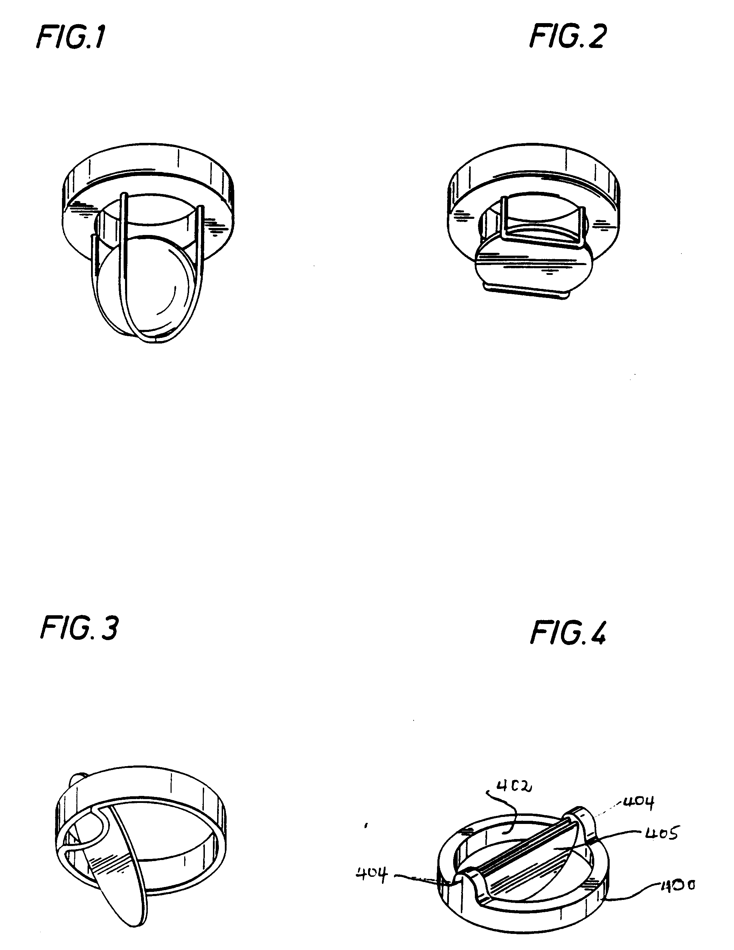

In its simplest form, a synthetic cardiac valve includes a valve body for affixing the valve to the body tissue and through which blood flows, and a flow control element for allowing or blocking off blood flow. For instance, Figure 4 shows a typical bileaflet valve having a valve body that includes a ring-like housing 400 with an inner ring 402 that has two flanges 404 each containing two slots for receiving hinges attached to leaflets. The flow control element of this valve comprises two leaflets 405, in the approximate shape of half discs, with hinge elements attached at diametrically opposite ends. These hinge elements fit within apertures or slots in the flanges 404 of the inner ring 402 and are able to rotate through less than 180°C in these apertures. Thus, in operation, the flow control elements are in the position shown in Figure 4 with the valve open with blood flowing from top to bottom. When blood flow reverses and flows from bottom to top, the bileaflets 405 pivot about their hinges to close the apertures in the ring-like valve body. Consequently, there is a significant amount of movement about the hinge elements and slots where microfretting wear might be initiated. Furthermore, the bileaflet half disc flow control elements 405 may impinge upon the inner ring 402 of the valve body, thereby leading to cavitation or impact-induced wear.

The invention provides heart valves of various designs, exemplified in Figure 1-4, each comprising parts subject to impact and wear that are fabricated from Ti-Nb-Zr alloy. More recent designs (not shown in the Figures) include concave bileaflet and trileaflet designs which are intended to improve blood flow. The heart valves are preferably subjected to a hardening process, such as oxygen or nitrogen diffusion hardening to produce a harder Ti-Nb-Zr surface that is resistant to microfretting wear at hinge points and impact wear at those locations where a flow control element impacts the valve body. Consequently, the invention valves have a longer cycle life than the currently used St. Jude, Omniscience, Starn-Edwards, Medtronic-Hall, or Baruah valves. Indeed, as mentioned before, the Baruah valve is currently fabricated of zirconium or zirconium alloys and would therefore be subject to relatively rapid wear because of the relative softness of zirconium and its alloys. The use of Ti-Nb-Zr alloy compositions also provides a low thrombus, blood-compatible surface favourable in reducing the incidence of blood clots.

### Artificial Hearts/Ventricular Assist Devices

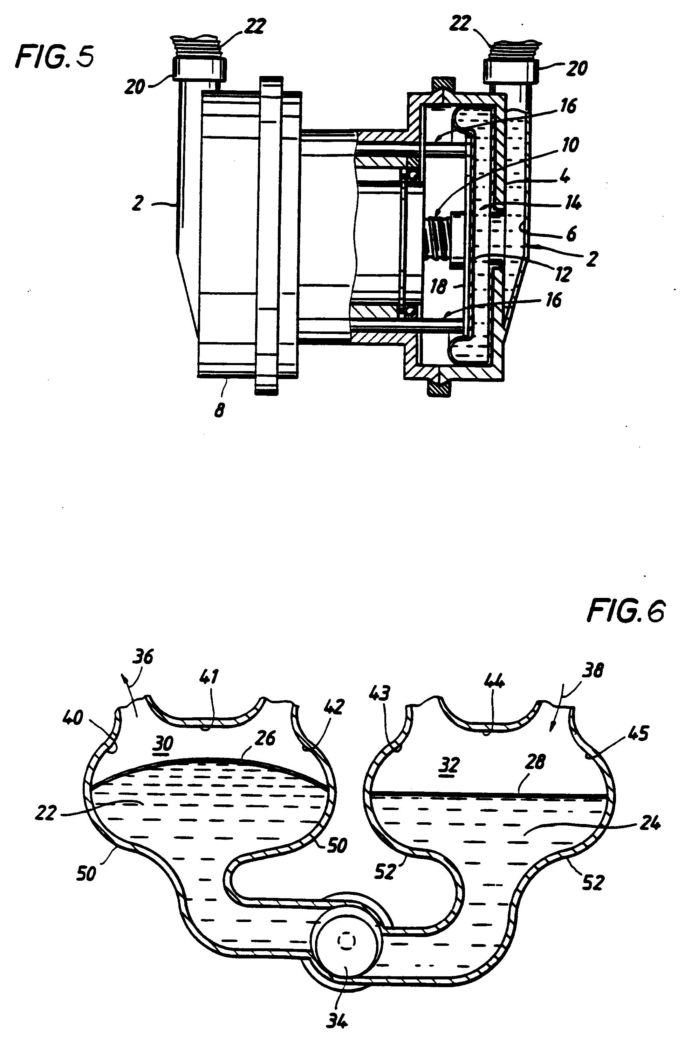

Figure 5 is illustrative of the Penn State design which incorporates a stainless steel roller screw 10 positioned between two flexible diaphragm blood pumps (right side pump 12 is shown, the other is within shell 8). A high speed, low torque brushless DC motor causes the roller screw 10 to turn thereby moving the roller screw shafts 16 linearly back and forth. To each end of the guide shaft 16 is attached a pusher plate 18. When the pusher plate 18 in the right side pump moves backward, blood is drawn into the pump space 14. At the same time, the pusher plate in the corresponding left side pump moves towards the left pushing blood out of its pump space. In this type of pump, the pusher plates 18 act against a flexible membrane 12, which is in contact with blood, and which can be inflated on the pump suction stroke and deflated on pump discharge stroke. The pusher plates are driven by the roller screw 10 and, thus, according to the invention, six revolutions of roller screw 10 are required for a full stroke with a motor speed of about 3,000 RPM. While planetary rollers are inserted between a roller screw nut and roller screw 10 to give rolling, not sliding contact and to spread mechanical load over many contact points, and while the entire roller screw system is improved by the use of the invention components. Thus, roller screw 10 and the roller screw nut with which it is in rolling contact are both fabricated of Ti-Nb-Zr alloy and the surfaces are hardened or coated with a hard, tightly adherent coating. Further, the surfaces of the pump nozzles 2 shown as 4 and 6, connecting elements 20 and conduits 22, into and from which blood is continuously being pumped, is fabricated from Ti-Nb-Zr to reduce adverse reaction with blood tissue on those surfaces presented to blood components to minimize the potential for the formation of thrombus and blood clots.

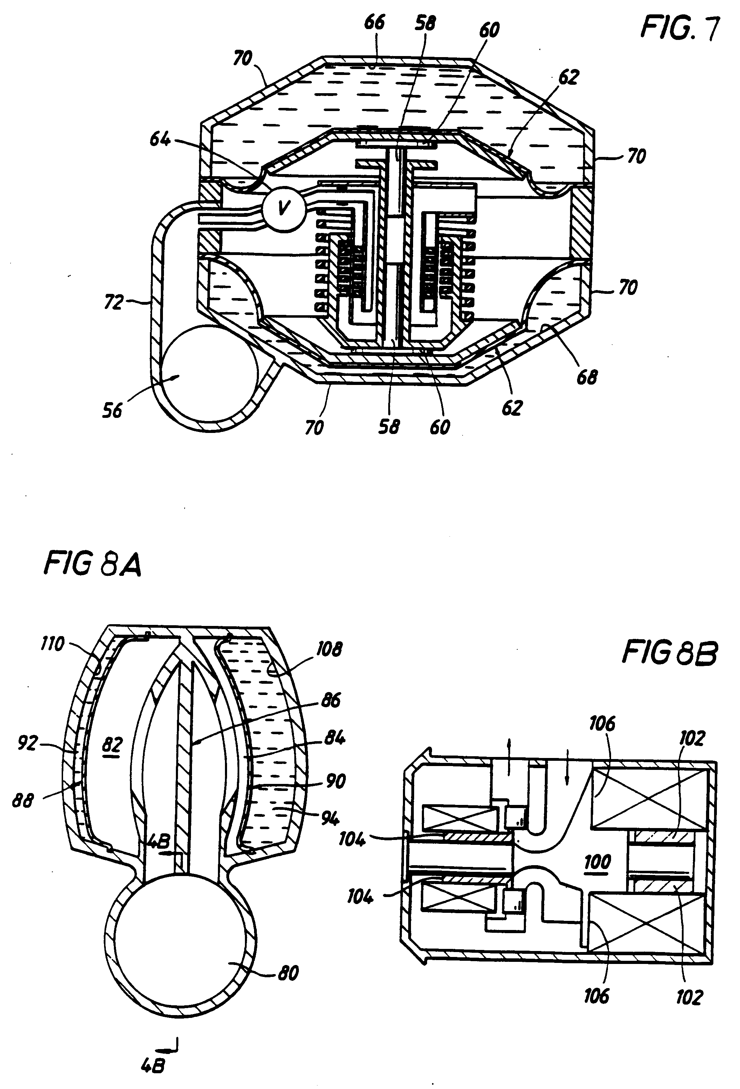

Figure 6 is a schematic cross sectional diagram of the University of Utah electrohydraulic heart. This heart includes shells 50 and 52 with a pump motor 34 interposed between them. In this type of heart, a motor 20 is used to pressurize silicone oil in regions 22 and 24 on the undersides of flexible diaphragms 26 and 28, respectively, to move blood in and out of the chambers 30 and 32 above the flexible diaphragms. For example, when motor 34 pressurizes silicone oil into chamber 22, then flexible diaphragm 26 expands upward and outwardly to push blood flow out of chamber 30 in direction 36. At the same time, silicon oil flows out of chamber 24 towards chamber 22 thereby allowing flexible diaphragm 28 to assume a natural position, shown in Figure 2, and drawing blood into chamber 32 as shown from direction 38. Upon reversal of the direction of the bidirectional pump 34, the opposite effects are achieved.

Since the University of Utah pump is of a bidirectional design, and typically operates at speeds between 10,000 - 13,000 RPM in the high pressure direction and 5,000 - 8,000 RPM in the reverse, moving components of the pump are subject to microfretting and mechanical wear. Therefore, the invention components for the bidirectional axial flow pump 34 used in the University of Utah TAH design are fabricated from Ti-Nb-Zr alloy coated hardened or with a wear resistant, hard coating that is tightly adherent, to reduce wear of the high speed components. Further, surfaces 40, 41, 42, 43, 44 and 45 are in direct contact with blood and are made of Ti-Nb-Zr alloy to improve blood compatibility and reduce the potential for thrombus and blood clotting. Thus, the shells of the heart 50 and 52 are also fabricated of Ti-Nb-Zr alloy to reduce thrombogenesis.

Figure 7 is a schematic cross sectional illustration of the Cleveland Clinic TAH which utilizes a motor to turn a gear pump 56 which provides hydraulic pressure at about 100 psi to cause reciprocal movement of actuators 58 which in turn drive pusher-plates 60 that act on flexible diaphragms 62 to pump the blood. The actuators 58 operate slidingly within guide sleeve 64 so that wear on contact surfaces between actuator and sleeve may be expected. Further, the TAH has a flow reversing valve 64 with machine elements, such as bearing surfaces, subject to wear when the TAH is in use. Thus, TAH elements that are subject to wear and that may be advantageously fabricated of Ti-Nb-Zr alloys that are then surface hardened and/or coated with a hard, wear resistant, tightly adherent coating, include the guide sleeve 64, the actuators 58, the pump's gear elements and shaft and the rotary valve 64. Moreover, to reduce the risk of erosion damage to the pump from cavitation, the pump housing 72 may likewise be fabricated of Ti-Nb-Zr alloys. Finally, internal surfaces 66, 68 of the heart housing 70 are in direct blood contact. Thus it is desirable to fabricate housing 70 from Ti-Nb-Zr to reduce the risk of thrombogenesis.

Figures 8A and B are schematic diagrams of the Texas Heart Institute/Abiomed TAH design which utilizes a d.c. motor to drive a miniature centrifugal pump 80 that rotates at about 6,000 - 8,000 RPM. This pump 80 pressurizes hydraulic fluid alternately into chambers 82 and 84 separated by septum 86 and enclosed by flexible diaphragms 88 and 90 respectively. As fluid is pumped into chamber 82, diaphragm 88 expands into heart space 92 forcing blood from this space. At the same time, fluid is pumped from chamber 84 causing diaphragm 90 to relax and expanding heart space 94, drawing blood into the TAH. The hydraulic flow is reversed by a two-position 4-way rotating valve 100 of radial configuration for compactness. Rotary valve 100 rotates within sleeves 102 and 104 at high speed so that contacting surfaces between the valve 100 and these sleevesare subject to wear. Further, rotary valve 100 rotates against seals 106 and wear may be expected at the contacting surfaces of the seals and the valve 100.

Several components of the Texas Heart Institute/ Abiomed TAH may be fabricated according to the invention. Thus, high speed components of the centrifugal pump 80 subject to wear may be fabricated from Ti-Nb-Zr alloy and then surface hardened and/or coated with a tightly adherent, hard, wear resistant coating. Further, the rotary valve 100 itself and the surfaces of sleeves 102, 104 and seals 106 may be fabricated from Ti-Nb-Zr alloy then surface hardened or coated with an adherent, wear-resistant coating. Finally, the inner surfaces of the TAH 108, 110 may be fabricated from Ti-Nb-Zr alloys to improve blood compatibility and reduce the potential for thrombus and blood clotting.

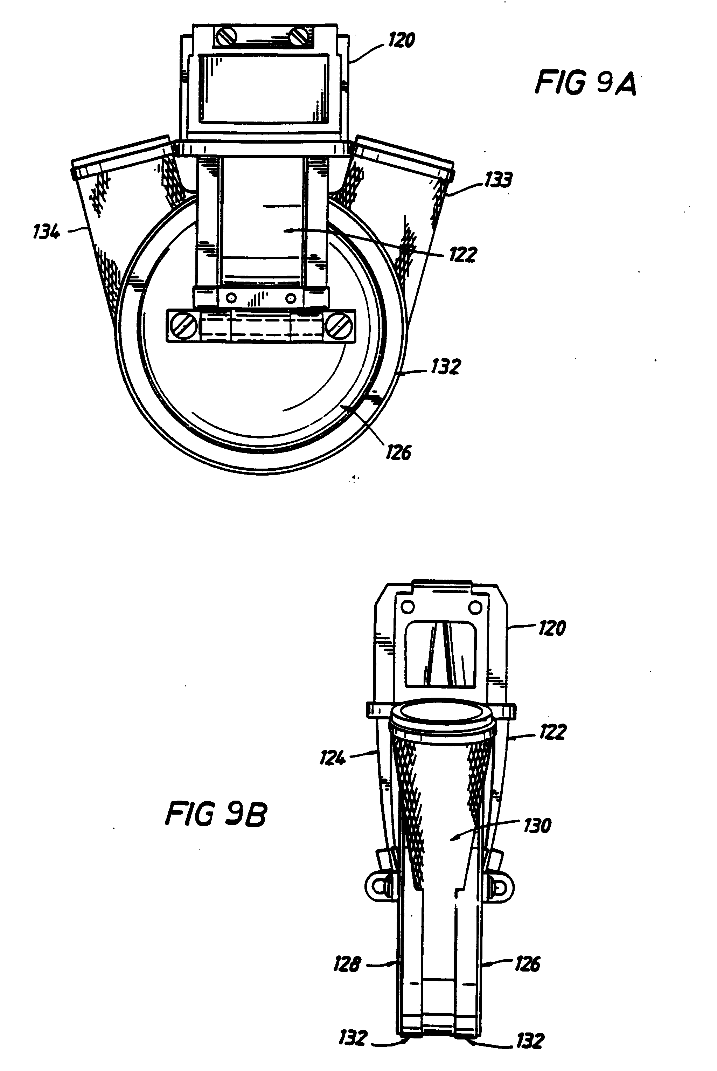

The Novacor designed VAD illustrated in Figures 9A and B have a solenoid mechanism 120 which sends energy through beam-springs 122, 124 that extend to the back of pump pusher plates 126, 128. The energy stored in the springs translates into linear motion of the plates which exerts force on the flexible blood sac 130. The blood sac 130 consists of a butyl rubber layer sandwiched between two layers of polyurethane Biomer. The blood sac 130 is supported within a cylindrical aluminium ring 132 that acts as a pump housing. The blood inflow 133 and outflow 134 ports are positioned tangentially on opposite sides of the housing to ensure straight-through blood flow. The ports are formed of an epoxy-impregnated Kevlar fabric shell that is integrated into the housing. The ports also encapsulate trileaflet inlet and outlet valves made from bovine pericardium tissue. When implanted into the body, fittings for attaching inflow and outflow valves to vascular conduits are bonded to a pump bulkhead, not shown, which also provides the framework for an encapsulating shell around the pump. This encapsulating shell also has provision for mounting the solenoid energy converter. The solenoid energy converter consists of two solenoid mechanisms, two lightweight titanium beam-springs, and an aluminium support structure. All of these metallic components would come into contact with blood components and body tissue. Therefore, the invention proposes that the titanium beam-springs be replaced with beam-springs of Ti-Nb-Zr alloy. Further, the aluminium support structure would likewise be replaced with a Ti-Nb-Zr alloy support structure that may optionally be hardened and/or coated with a hard coating.

Novacor has identified, in designing the solenoid, that "the challenge was coming up with something that would run for 100 million cycles a year, without requiring maintenance". O'Connor, Lee, "Novacor's VAD: How to Mend a Broken Heart", Mechan. Engr'g pp. 53-55 (Nov 1991). The invention components fabricated from Ti-Nb-Zr alloys then hardened or coated with hard, wear resistant coatings provide surfaces that are hard, microfretting wear resistant, biocompatible and blood compatible so that they would meet this goal. To further reduce friction and wear of wear surfaces of implant devices, a thin boron or silver surface layer can be applied as an overlay on the previously diffusion hardened Ti-Nb-Zr surface.

External mechanical hearts (EMHs) are used as a bridge to transplant. These hearts include the Jarvik-7 pneumatic heart and the more recent left-ventricular assist device, the Heartmate developed by Thermocardio Systems. In the Heartmate system, two tubes, one carrying air and the other electrical wire, pass from outside the body to an implanted blood pump. The pump is implanted in the abdomen and removes blood from the natural heart's left ventricle. This blood enters and exits the pump through 25 millimeter input and output valves made from chemically processed bovine tissue. The blood flows from the output valve through a dacron-wrapped polyurethane tube to the aorta. An electric motor mounted in the Heartmate's lower chamber actuates a flat-plate piston, which is bonded to a flexible polyurethane diaphragm. When the motor goes through one revolution, it turns a cam assembly that compresses the diaphragm, which pushes blood through the output valve. The operation of the pump is controlled by a microprocessor located in a shoulder bag which adjusts the heartbeat rate by changing the motor's current. According to the invention, the moving parts of the heartmate pump may be replaced with components fabricated from Ti-Nb-Zr alloys then hardened or coated with a hard coating to reduce mechanical wear, friction, and microfretting wear. Furthermore, those metallic components that come into contact with blood components, may also be replaced with Ti-Nb-Zr alloy components similarly coated to improve blood compatibility and reduce the risk of clot formation.

The gravest problem in the use of the pneumatic Jarvik-7 heart has been identified as the formation of blood clots. O'Connor, Lee, "Engineering a Replacement for the Human Heart", Mechan. Engr'g pp. 36-43 (Jul 1991). In 1990, the FDA withdrew the Jarvik system from clinical trials due to concerns over quality control during manufacture. The University of Utah made modifications to the design of the Jarvik heart to develop a new system called the "Utah 100" which has elliptical pump housings, as opposed to the spherical housings of the Jarvik-7. Further, the Utah 100 has redesigned junctions for joining the diaphragms within the ventricles to the housing. These changes are said to have resulted in an about 70% reduction in blood clot formation relative to the Jarvik-7 design. However, according to the invention, yet further reduction in blood clot formation may be obtained by fabricating moving parts and those metallic surfaces that contact blood components from Ti-Nb-Zr alloys and then hardening and/or coating these components with hard, wear resistant coating to increase blood compatibility and thrombus resistance, and to reduce abrasive wear, and reduce microfretting wear.

### Guide Wires and Catheters

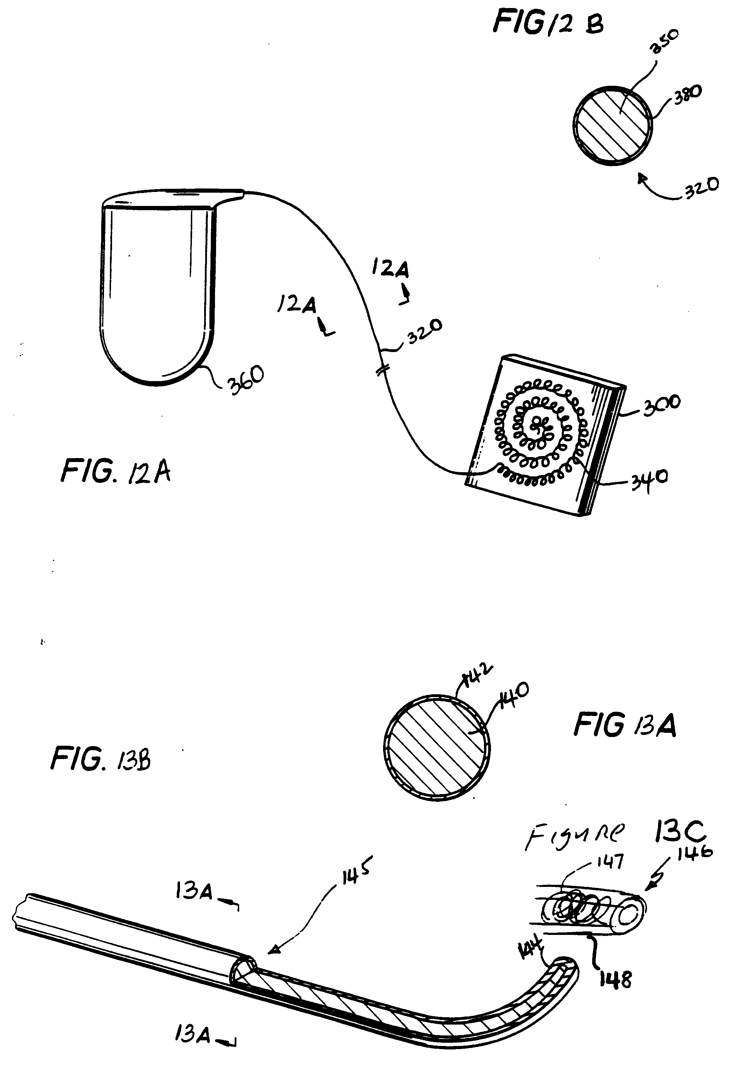

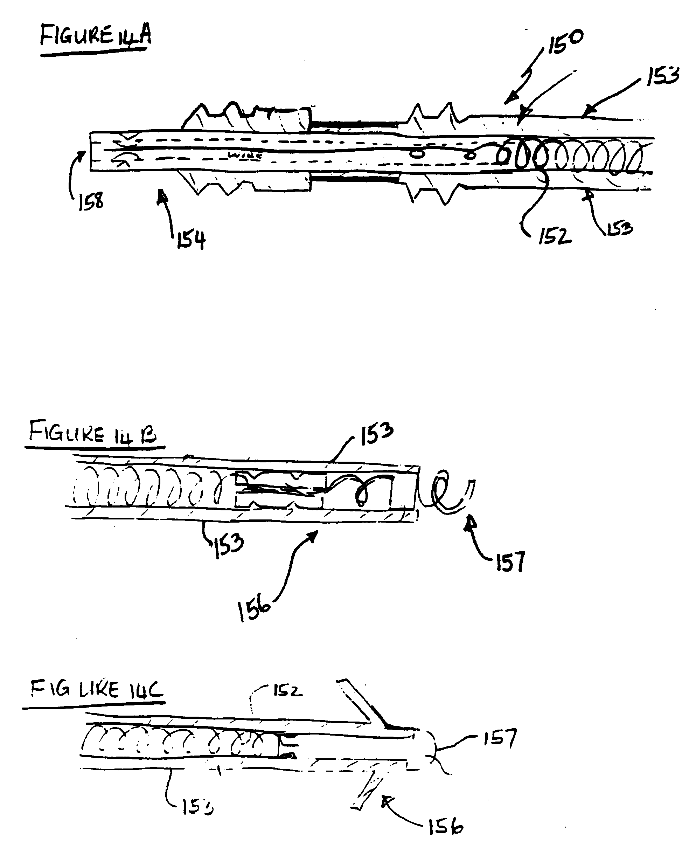

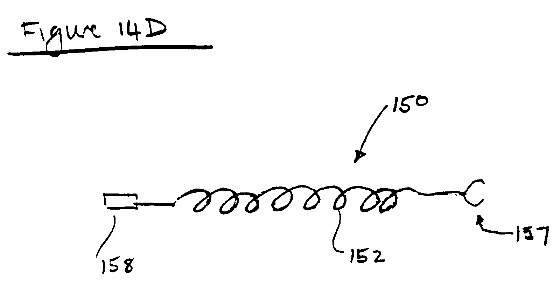

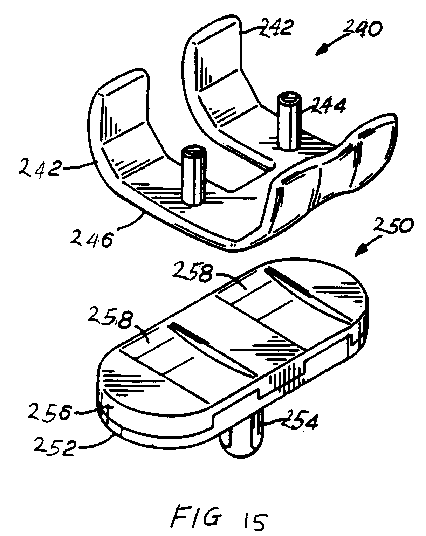

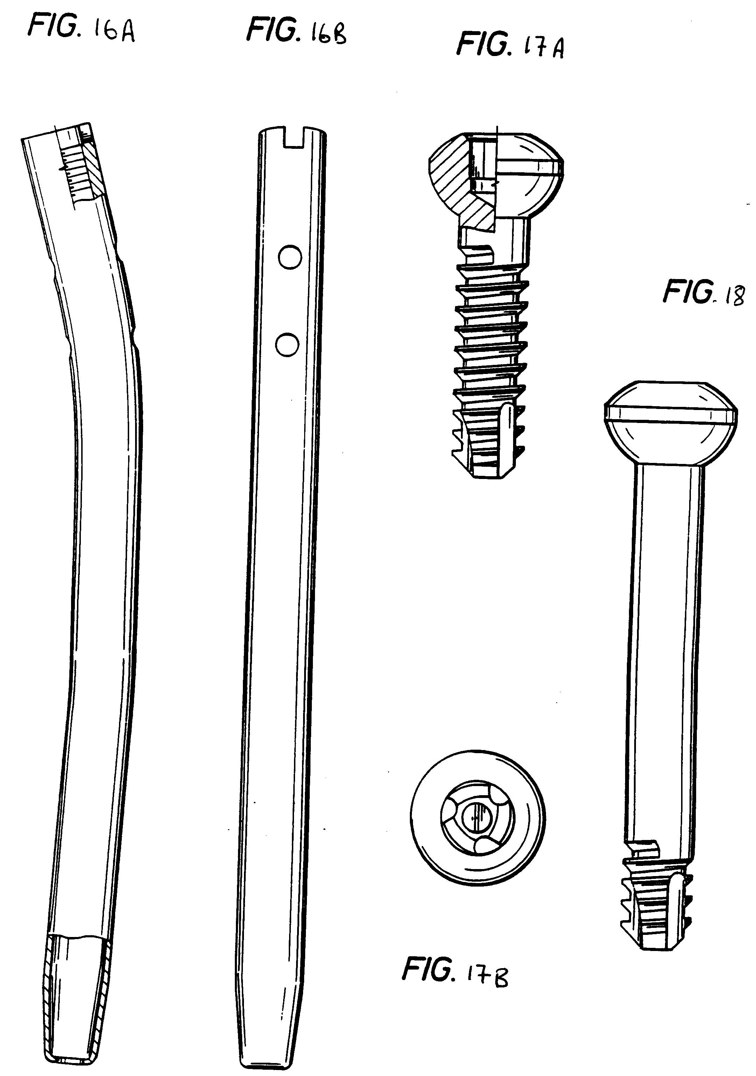

Figures 13A and B show, in partial cross section, the distal end of a guide wire fabricated according to the invention. The guide wire 145 has a core 140 of Ti-Nb-Zr alloy with a surface hardened coating 142 to reduce friction which may be further coated with a material that is bio- and hemocompatible and of low friction when in contact with the catheter wall or body tissue. The flexible catheter 146 through which the guide wire moves is also fabricated according to the invention and includes a coil 147 of low modulus titanium alloy which is generally encased by a polymer sheath 148 as shown schematically in Figure 13C. The guide wire in this case is equipped with a cutting tip 144, preferably also made of Ti-Nb-Zr alloy with a diffusion hardened surface optionally with a hard ceramic or lubricating coating. Since the guide wire is fabricated of metal, it is highly visible under x-rays, providing excellent radiopacity. Boron or silver surface layers may also be deposited on the diffusion hardened surfaces to further reduce friction and wear.

### Pacemakers and Electrical Signal Carrying Leads/Sensors

Pacemaker and other electronic leads are manufactured by several corporations, including Medtronic, which produces a range of pacemaker lead designs.

One of these designs is shown in schematic form in Figures 14A and B. The pacemaker lead body 150 has a centrally disposed metallic conductor 152 typically made of cobalt-nickel alloy, such as MP35N (Trade Mark). This conductor 152 is usually made up of several strands of wire, each having a diameter of about 0.15-0.20 mm. The conductor 152 is covered by an insulative, protective polymer sheath 153 so that the elongate body 150 of the pacemaker lead has an overall diameter ranging from about 2.2 to about 3 mm. The pacemaker has a first end 154 with an electrode 158 for connecting to a pulse generator and a second end 156 with an electrode 157 for contacting heart muscle. An alternative embodiment is shown in Figure 14C. As supplied, these two ends are covered with protective polyurethane caps which can be removed for installation of the pacemaker. In order to prevent electrical interference with the conductor 152, a polymeric insulative sleeve 153 is disposed over the entire pacemaker lead body 150, with the exception of the exposed electrodes 157 for contacting heart muscle and the contact electrode 158 for engaging with the pulse generator that houses the electronics and power pack for the pacemaker. As explained before, the organic polymeric sheath compositions, typically polyurethane, can slowly degenerate in the body causing problems, not only due to potential deterioration of electrical insulation and interference with electrical signals but also because of potentially toxic products of degradation.

The invention provides, as shown in Figure 14C, a pacemaker wherein the conductor 152 is fabricated from a Ti-Nb-Zr alloy that is coated with a tightly adherent, low friction, bio- and hemocompatible coating, with the exception of the electrode for contacting heart muscle 157, and the electrode 158 at the other end of the lead for engaging the pulse generator. The coatings can be formed by in situ oxidation or nitriding of the Ti-Nb-Zr to produce an electrically insulative surface layer of from about 0.1 to about 3 microns in thickness, preferably less than about 0.5 microns in thickness. This process can be carried out at the same time the material is age-hardened. Alternatively, an insulative inert ceramic coating can be applied by conventional CVD or PVD methods either on the original Ti-Nb-Zr alloy surface or onto the diffusion hardened Ti-Nb-Zr surface. For these overlay coatings, the thickness can be as great as 20 microns. The overlay coatings include ceramic metal oxides, metal nitrides, metal carbides, amorphous diamond-like carbon, as detailed above. The electrical signal conductor 152 can comprise either a single wire or multiple wires. Exposed Ti-Nb-Zr metallic ends of the wire or wires are preferably connected directly to a pulse generator thereby avoiding the necessity for a weld or crimp to attach an electrode to the conductor which may result in local galvanic corrosion or physically weakened regions. Further, since the coatings provide a natural protective insulative surface, the use of a coiled construct could be avoided by using only a preferred single-strand, non-coiled low modulus Ti-Nb-Zr metallic wire construct for the conductor 152. This will also eliminate the need for stiff guide wire. Finally, the overall diameter of the pacemaker lead body 150 could be reduced considerably from the range of about 2.3 - 3 mm for current commercially available leads to about 0.2 - 1 mm. Optionally, the leads of the invention may be covered with a polymeric sheath.

### Stents

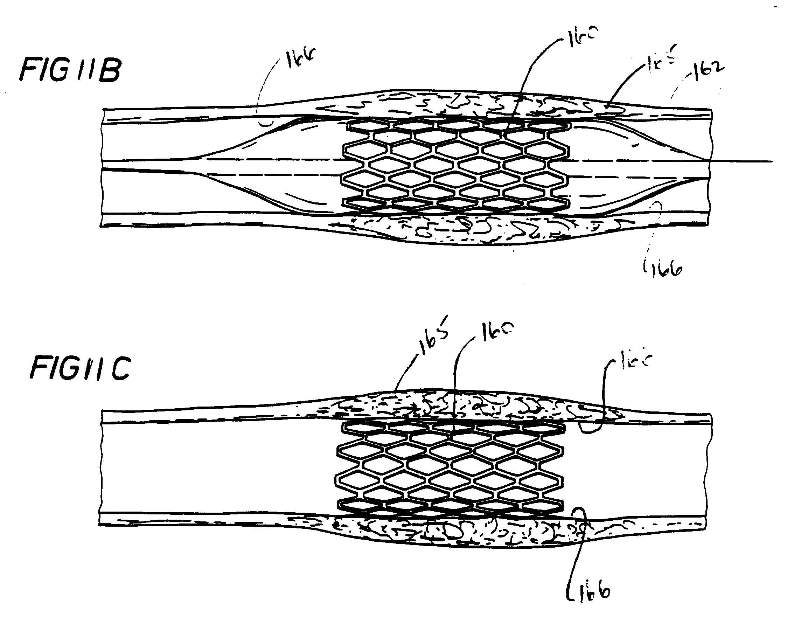

Figure 11A shows a schematic of an expandable stent 160, in non-expanded state, positioned on the distal end of a balloon expandable segment 162 of a guide wire 164. The stent is fabricated from Ti-Nb-Zr alloy and is designed so that it can be collapsed over a balloon segment of a balloon catheter. When the stent is in position, within segment of a tubular conduit 165 in the body, a blood vessel for example, to be propped open, the balloon 162 is expanded thereby expanding the stent 160 radially outward up to the blood vessel wall 166 so that means for gripping soft tissue, such as barbs (not shown), on the outer surface of the stent 160 engage and grip blood vessel tissue to anchor the stent 160 in position as shown in Figure 11B. The balloon 162 is then collapsed and removed leaving the stent in place as shown in Figure 11C. In this way, the blood vessel is permanently propped open. Urinary, gastrointestinal, and other stent applications are also provided using Ti-Nb-Zr alloy.

### Grafts

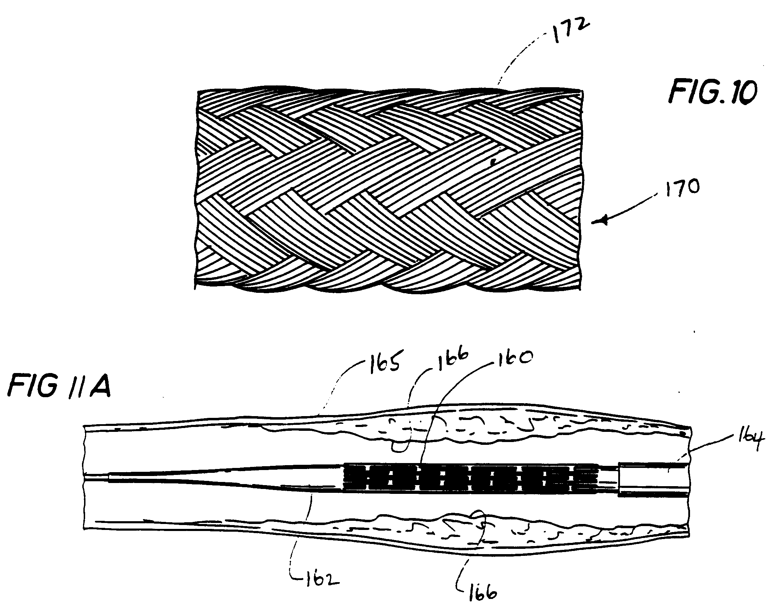

Figure 10 is a representative sketch of a side view of a substantially tubular vascular graft 170 sized to graft onto a blood vessel and made of woven low modulus Ti-Nb-Zr wires 172. While the graft shown is made of woven wires of Ti-Nb-Zr, the graft can also be fabricated from a cylindrical tubing of this alloy. The graft can be fabricated from Ti-Nb-Zr alloy in the lower modulus cold worked condition, or in the slightly higher modulus aged condition with optional surface hardening. Additionally, protein, antibiotic, anti-thrombic, and other surface treatments may be employed to further improve the biocompatibility and clinical performance.

### Defibrillators

Figures 12A and B show a defibrillator including a flexible silicone polymeric patch 300 with a coil of conductive wire 320 (typically titanium, stainless steel, or cobalt-nickel-chromium) on the side of the silicone patch 300 that will contact muscle tissue. When in place in the body, the lead wire 320 that carries power to the coil 340 extends out of the body (through the skin) and is electrically connected to a power source contained in a protective container 360. According to the invention, the lead wire 320 is fabricated with an electrically conductive core 350 of Ti-Nb-Zr alloy and is coated with an adherent electrically insulative coating 280, such as metal oxides, carbides, or nitrides, or with amorphous diamond-like carbon as shown in exaggerated detail Figure 12B. This coating electrically insulates the lead wire from electrical contact with surrounding body tissue while also protecting the metallic core from corrosion and attack by body fluids, as described previously, for the pacemaker lead. Elimination of the polymer coating results in the elimination of potentially toxic products of gradual degradation of the polymer and also the consequent shorting the system when the insulative coating is breached.

### The Hardened Surfaces

The oxygen or nitrogen diffusion hardened surface of the alloy implants may be highly polished to a mirror finish to further improve blood flow characteristics. Further, the oxide- or nitride-coated surfaces may be coated with substances that enhance biocompatibility and performance. For example, a coating of phosphatidyl choline, heparin, or other proteins to reduce platelet adhesion to the surfaces of the implant, or the use of antibiotic coatings to minimize the potential for infection. Boronated or silver-doped hardened surface layers on the implant reduces friction and wear between contacting parts of heart valves, prosthetic artificial hearts, ventricular assist devices, and other contacting parts in the invention cardiovascular implants. Additionally, amorphous diamond-like carbon, pyrolytic carbon, or other hard ceramic surface layers can also be coated onto the diffusion hardened surface to optimize other friction and wear aspects. The preferred diffusion hardened surface layer described in this application provides a hard, well-attached layer to which these additional hard coatings with respect to hardness. Other, conventional methods of oxygen surface hardening are also useful. Nitriding of the substrate leads to a hardened nitride surface layer. Methods of nitridation known in the art may be used to achieve a hard nitride layer.

Regardless of how a Ti-Nb-Zr alloy implant's surface is hardened, the friction and wear (tribiological) aspects of the surface can be further improved by employing the use of silver doping or boronation techniques. Ion-beam-assisted deposition of silver films onto ceramic surfaces can improve tribiological behaviour. The deposition of up to about 3 microns thick silver films can be performed at room temperature in a vacuum chamber equipped with an electron-beam hard silver evaporation source. A mixture of argon and oxygen gas is fed through the ion source to create an ion flux. One set of acceptable silver deposition parameters consists of an acceleration voltage of 1 kev with an ion current density of 25 microamps per cm². The silver film can be completely deposited by this ion bombardment or formed partially via bombardment while the remaining thickness is achieved by vacuum evaporation. Ion bombardment improves the attachment of the silver film to the Ti-Nb-Zr alloy substrate. Similar deposition of silver films on existing metal cardiovascular implants may also be performed to improve tribiological behaviour, as well as antibacterial response.

An alternative method to further improve the tribiological behaviour of Ti-Nb-Zr alloy surfaces of cardiovascular implants is to apply boronation treatments to these surfaces such as commercial available boride vapour deposition, boron ion implantation or sputter deposition using standard ion implantation and evaporation methods, or form a boron-type coating spontaneously in air. Boric Acid (H₃BO₃) surface films provide a self-replenishing solid lubricant which can further reduce the friction and wear of the ceramic substrate. These films form from the reaction of the B₂O₃ surface (deposited by various coventional methods) on the metal surface with water in the body to form lubricous boric acid. Conventional methods that can be used to deposit either a baron (B), H₃BO₃, or B₂O₃ surface layer on the cardiovascular implant surface include vacuum evaporation (with or without ion bombardment) or simple oven curing of a thin layer over the implant surface. The self-lubricating mechanism of H₃BO₃ is governed by its unique layered, triclinic crystal structure which allows sheets of atoms to easily slide over each other during articulation, thus minimzing substrate wear and friction.

Additionally, surfaces (metal or coated) of all the cardiovascular and medical implants discussed may optionally be coated with agents to further improve biological response. These agents include anticoagulants, proteins, antimicrobial agents, antibiotics, and the like medicaments.

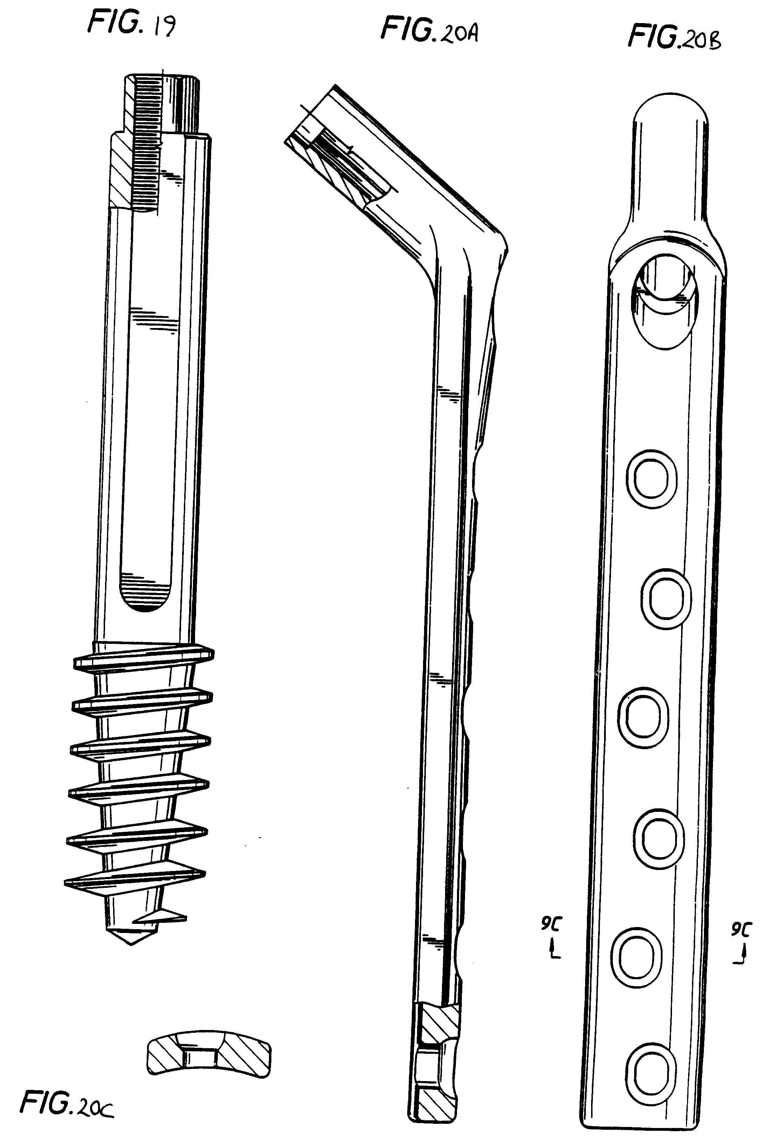

The titanium alloys are also useful in the manufacture of medical implants, and possess the characteristics of high strength, low modulus of elasticity, corrosion resistance to body fluids and tissue, and are free from any potentially toxic elements. Thus, the alloys are especially useful in the fabrication of bone plates (Figures 20A,B,C), intramedullary rods (Figures 16A,B), compression hip screws (Figure 19), spinal implants, modular knee joints (Figure 15), and the like. Typical modular knee joints as shown in Figure 15 include a femoral component 240 and a tibial component 250. The femoral component includes condyles 242 which provide articulating surfaces and pegs 244 for affixing to the femur. The tibial component 250 includes a tibial base 252 with a peg 254 for mounting the base onto the tibia. A tibial platform 256 is mounted atop the tibial base 252 and is supplied with grooves 258 that cooperate with the condyles 242. The tibial platform 256 is frequently made of an organic polymer (such as ultra-high molecular weight polyethylene) but the tibial base 252 and femoral component 240 are fabricated of metal. The invention provides tibial bases and femoral components of the above-described titanium-niobium-zirconium alloys.

The preferred titanium alloys for medical implants for use other than in the cardiac system include: (1) from about 10 to about 20 wt% niobium or from about 35 to about 50 wt% niobium, and (2) optionally up to 20 wt% zirconium.

The most preferred inventive alloy for medical use outside the cardiac system contains titanium as the major component comprising about 74 wt% of the alloy in combination with about 13 wt% of zirconium and 13 wt% of niobium.

While tantalum may be substituted for niobium to stabilize the β-phase titanium, niobium is the preferred component due to its effect of lowering the elastic modulus of the alloy when present in certain specific proportions. Other elements are not deliberately added to the alloy but may be present in such quantities that occur as impurities in the commerically pure titanium, zirconium, niobium or tantalum used to prepare the alloy and such contaminants as may arise from the melting (alloying) process. Filler materials, such as non-toxic tantalum, could also be added to reduce the β-transus (stabilze β) and improve strength as long as the relatively low modulus of elasticity (less than about 90 GPa) of the base alloy is not significantly affected.

Based upon the foregoing, it is apparent that the titanium proportion of certain embodiments of the invention alloy could be less than 50 wt%. Nevertheless, these alloys are, for purposes of the specification and claims, referred to as "titanium alloys". For example, a titanium alloy may contain 20 wt% zirconium and 45 wt% niobium with only 35 wt% titanium.

While the as-cast or powder metallurgically prepared alloy can be used as an implant material or for other applications, it can optionally be mechanically hot worked at 600-950°C. The hot working process may include such operations as extrusion, hammer forging, bending, press forging, upsetting, hot rolling, swagging, and the like. After the final hot working step, the alloy should be cooled rapdily, as for instance, by water quenching. Slower rates of cooling, as by air convection cooling, are not recommended and are not effective in producing the high strength, low modulus alloy suitable for use as a medical implant. After cooling, it can then be reheated, preferably gradually over a period from about 0.5 to 10 hours, more preferably 1.5 to 5 hours to a maximum temperature of about 700°C, preferably about 500°C. Then, the implant material is maintained at this temperature for from about 0.25 to 20 hours, preferably for from about 2 to about 8 hours, more preferably for about 6 hours. This process, by a phenomenon called precipitation strengthening, is responsible for the high strength of the alloy in the hot worked, quenched and aged condition described above.

In the specification and claims, the term "high strength" refers to a tensile strength above about 700 MPa, preferably above about 800 MPa.

The term "low modulus" as used in the specification and claims refer to a Young's modulus below about 90 GPa.

In titanium alloys, the niobium (or tantalum, if this element is added) acts to stabilize the β-phase since it is a β-isomorphous phase stabilizer. This results in a lower β-phase transus temperature and improved hot workability.

Niobium, in particular, when present in preferred quantities of from about 6 to about 10 atomic percent (most preferably about 8 atomic percent) or in an alternative preferred range of from about 22 to 32 atomic percent, produces a low modulus composition when alloyed with titanium. Deviation from these ranges of niobium concentration tends to increase the elastic modulus. In weight percent terms, these preferred compositional ranges of niobium in the titanium-zirconium alloy translate to about 10 to about 20 wt% and about 35 to about 50 wt%.

Titanium alloys containing about 13 wt% niobium correspond to those having about 8 atomic percent niobium. Thus, the Ti-13Nb-13Zr alloy is believed to identify an optimal low modulus, titanium alloy composition.

As previously mentioned, tantalum may be substituted for niobium to stabilize the β-phase, but niobium is preferred due to its effect in reducing the elastic modulus. Substitution with zirconium can improve strength.

Whereas the niobium proportion is critical to obtain the desired low modulus property, the zirconium proportion is not as critical. It is desirable to maintain the proportion of zirconium at less than about 20 wt% but higher proportions are also useful.

Zirconium, it is believed, is capable of stabilizing both α and β-phase titanium alloy, but acts by being in solution in the alloy as a β-stabilizer by slowing the transformation process in the inventive alloy. It is further believed that the larger ionic radius of zirconium (35% larger than that of titanium) helps to disrupt ionic bonding forces in the alloy resulting in some reduction in the modulus of elasticity.

In order to effect the translation to the β-phase (which is not essential to produce the high strength, low modulus alloy implants of the invention), the alloy may be treated by heating to above the β-transus temperature, eg. to about 875°C, for about 20 minutes. Lower temperatures above the β-transus may also be used. The β-phase may also be induced by heating to higher temperatures for shorter periods of time. The critical factor for transition to the β-phase is heating to at least about the β-transition temperature, which is about 728°C for Ti-13Zr-13Nb, for a period of time sufficient to obtain a substantial conversion of the titanium alloy to the β-phase prior to cooling to room temperature. Conversion of the alloy to the β-phase and cooling may be effected before, during or after shaping for implantation and sintering of a porous metal coating, whichever is most convenient.

It should be noted that heating to the above the β-transus and hot working at such elevated temperature while converting most or all of the alloy to the β-phase, is not essential to obtain the desired high strength and low modulus. Indeed, the alloy may be heated to a temperature as low as about 100°C below the β-transus, and hot worked at this lower temperature to achieve high strength and low modulus, after rapid quenching and aging, without complete transformation to the β-phase. The region immediately below the β-transus temperature is called the α + β region. Hot working may be performed after heating to this α + β region and, possibly, even temperatures below this region, ie. temperatures as low as about 100°C below the β-transus.

The β-transus temperature of the most preferred Ti-13Nb-13Zr alloy is about 728°C. The alloy may be heated to above the β-transus, eg. about 800°C, for forging. Other intermediate temperatures may also be used, but at temperatures lower than about 600°C forging may be difficult because of the poorer formability of the alloy at these low temperatures.

The machining, casting or forging of the alloy into the desired implant shape may be carried out by any of the conventional methods used for titanium alloys. Further, implants could be pressed from the powdered alloy under conditions of heat and pressure in pre-forms in the shape of the desired implant. Conventional sintering and hot isostatic pressure treatments can be applied.

While the alloy provides a non-toxic prosthesis material, it may yet be desirable for other reasons, such as micro-fretting against bone or polyethylene bearing surfaces, to coat the metal surface. In this event, the surface may be coated with an amorphous diamond-like carbon coating or ceramic-like coating such as titanium nitride or titanium carbide, or the oxide, nitride or carbide of zirconium, using chemical or plasma vapor deposition techniques to provide a hard, impervious, smooth surface coating.

Alternatively, a coating may be formed in situ on the shaped alloy by exposure to air, oxygen, and/or nitrogen at elevated temperatures to oxidize or nitride the surface of the alloy to a desired depth. Typically these coatings, resulting from the diffusion of oxygen or nitrogen into the metal surface, are up to about 100µ thick or greater. These in situ coatings are tightly adherent and more wear resistant than the metallic alloy surface. Coatings are therefore especially useful if the alloy is subjected to conditions of wear, such as, for instance, in the case of bearing surfaces of knee or hip prostheses.

Methods for providing hard, low-friction, impervious, biocompatible amorphous diamond-like carbon coatings are known in the art and are disclosed in, for example, EPO patent application 302 717 A1 to Ion Tech and Chemical Abstract 43655P, Volume 101 describing Japan Kokai 59/851 to Sumitomo Electric, all of which are incorporated by reference herein as though fully set forth.

Further, the metal alloys may be hardened by interstitial ion implantation wherein the metal surface is bombarded with the ions of oxygen or nitrogen, and the like. The metal retains a metallic-appearing surface but the surface is hardened to a depth of about 0.1µ. The metals may also be surface hardened by internal oxidation, as described in our copending US Serial No.832,735, filed 7 February 1992.

Implants fabricated from the inventive alloy may be supplied with a porous bead, powder, or wire coating of titanium alloy of the same or different composition including pure titanium to allow stabilization of the implant in the skeletal structure of the patient after implantation, by bone ingrowth into the porous structure. Such porous structures are sometimes attached to the implant surface by sintering. This involves heating the implant to above about 1250°C. The mechanical properties of titanium alloys can change significantly due to substantial grain growth and other metallurgical factors arising from the sintering process. Thus, after sintering to attach the porous coating, it is preferred that the Ti-13Zr-13Nb implant be reheated to about 875°C (or above the β-transus) for 20-40 minutes then quenched before being aged at about 500°C for about 6 hours to restore mechanical properties. If quenched adequately from the sintering temperature, it may be possible to go directly to the aging process.

The following examples are intended to illustrate the invention as described above and claimed hereafter and are not intended to limit the scope of the invention in any way. The aging temperature used in the examples is determined to be acceptable, although not necessarily optimal.

### Example 1

An alloy including, by weight, 74% titanium, 13% niobium and 13% zirconium, was hot rolled at a temperature in the range 825-875°C to 14mm thick plate. The plate was cooled to room temperature then reheated to 875°C where it was maintained for 20 minutes and then water quenched to room temperature. The β-transus for this alloy was about 728°C as compared to about 1000°C for Ti-6A1-V. The mechanical properties of the heat-treated, quenched Ti-Zr-Nb alloy, which has an acicular transformed β-structure, are shown in Table I.

**TABLE I**

| Mechanical Properties of Ti-13Zr-13Nb As water Quenched from above β-Transus Temperature | |
|---|---|
| Tensile Strength | 710 MPa |
| Yield Strength | 476 MPa |
| Elongation | 26% |
| Reduction in Area | 70% |
| Young's Modulus | 62 GPa |
| Rockwell C Hardness | 18-19 |

### Example 2

The heat-treated, quenched Ti-Zr-Nb alloy of Example 1 was aged by heating at 500°C for 6 hours. The mechanical properties of this aged alloy are shown in Table II.

**TABLE II**

| Mechanical Properties of Quenched Ti-13Zr-13Nb Aged 500°C for Six Hours | |
|---|---|
| Tensile Strength | 917 MPa |
| Yield Strength | 796 MPa |
| Elongation | 13% |
| Reduction in Area | 42% |
| Young's Modulus | 76.6 GPa |
| Rockwell C Hardness | About 29 |

### Example 3

Samples of the alloy of Example 1 were sintered at about 1250°C to attach a porous titanium bead coating of the type shown in Figure 1. The bead-coated alloy samples were then reheated to 875°C and maintained at this temperature for 40 minutes before being water-quenched. A group of six samples were aged at 500°C for 6 hours and the mechanical properties of aged and non-aged samples (three each) were tested and shown in Table III.

**TABLE III**

| Mechanical Properties of Ti-13Zr-13Nb Alloy Following Bead Sintering, Reheating to 875°C, and Water Quenched | | |
|---|---|---|
| As-Quenched | (Avg) | Aged (500°C Six Hours) |
| Tensile Strength | 664 MPa | 900 MPa |
| Yield Strength | 465 MPa | 795 MPa |
| Elongation | 20% | 4% |
| Reduction Area | 46% | 9% |
| Young's Modulus | 61.8 GPa | 74.7 GPa |

Note that the sintering treatment can significantly alter the mechanical properties, particularly ductility. Thus, an alloy acceptable for a particular application in unsintered form may not necessarily be effective in that application following a high-temperature sintering treatment often used to attach a porous titanium coating. To minimize these effects, lower temperature diffusion bonding methods can be used in which a temperature near the β-transus may be effective. Alternatively, pre-sintered porous metal pads can be tack-welded to the implant. Yet another alternative is to apply the porous coating by a plasma-spraying method which does not expose the bulk of the material to high temperature.

### Example 4

A comparison of the elastic modulus, tensile strength and yield strength of the Ti-13Zr-13Nb invention alloy with those of known alloys, composites and cortical bone, are summarized in Figures 2 and 3. Al₂0₃ and Zr0₂ refer to ceramics while C/PEEK refers to carbon reinforced polyetheretherketone composite and C/PS refers to a carbon reinforced polysulfone composite. All the mechanical property data of Figures 2 and 3 were obtained from literature sources except for the data pertaining to the invention alloy which were measured using standard ASTM tensile testing techniques. It is significant that the Ti-13Zr-13Nb invention alloy has an elastic modulus similar to carbon fibre reinforced composites and closer to that of bone than the other metals (Figure 2) while at the same time possessing a strength comparable to or better than other metals (Figure 3).

### Example 5

A sample of Ti-18Zr-6Nb was sintered to attach a porous metal coating. Thereafter, the sintered alloy was reheated to 875°C, ie. above the β-transus, and water quenched. The properties of the as-quenched alloy are shown in Table IV. The sample was then aged at 450°C for 3 hours and tested. These results are also shown in Table IV.

As compared to the Ti-13Zr-13Nb alloy of Example 3, this alloy's modulus of elasticity is not as low but is still lower than that of Ti-6A1-4V. Further, the Ti-18Zr-6Nb alloy has a relatively low β-transus, about 760°C, compared to that of Ti-6A1-4V which is about 1000°C.

**TABLE IV**

| Mechanical Properties of Ti-18Zr-6Nb Following A High Temperature Sintering Treatment, Reheating to 875°C, and Water Quenching and Aging | | |
|---|---|---|
| | As-Quenched | Aged 450°C, 3 Hrs |
| Tensile Strength | 807 MPa | 876 MPa |
| Yield Strength | 659 MPa | 733 MPa |
| Elongation | 8% | 8% |
| Reduction in Area | 26% | 28% |
| Elastic Modulus | 85.2 GPa | 86.8 GPa |

Note that because of the less than optimum niobium content, the elastic modulus is not as low as the previous example. Thus, proper selection of niobium content is important for optimizing the low elastic modulus. However, the presence of zirconium helps to keep the elastic modulus at an acceptably low level (less than about 90 GPa).

### Example 6

The effect of aging conditions on Ti-13Zr-13Nb and Ti-18Zr-6Nb was investigated. Separate samples of each alloy were air-cooled or water-quenched from above the β-transus, aged at 500, 450, 400 and 350°C for up to 6 hours then air cooled. The results are recorded in Figure 4.

### Example 7

Forgings of Ti-13Nb-13Zr were prepared at temperatures of 800 and 680°C. These forgings were either water quenched or air cooled from the forging temperature and their mechanical properties were determined:

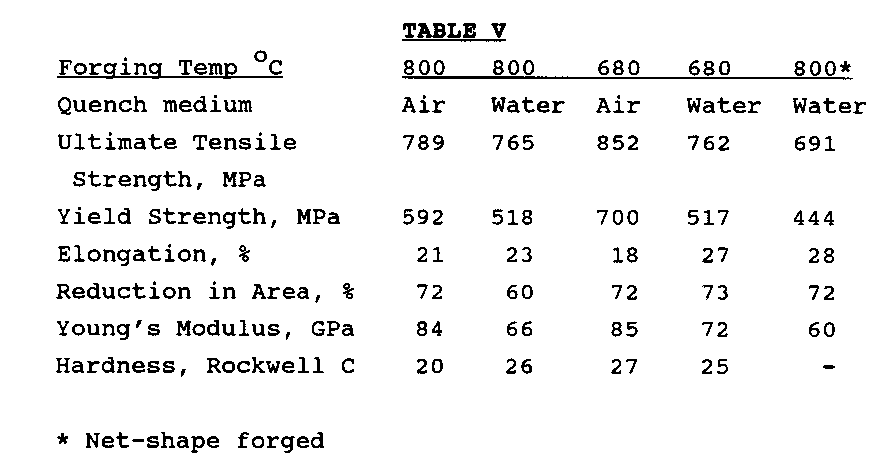

Forgings of 19mm minimum diameter Ti-13Nb-13Zr, which had been air cooled after forging, were heat treated by aging at 350 to 500°C for from 1.5 to 6 hours. The mechanical properties of these forgings were as follows:

**TABLE VA**

| Forging Temp, °C | 800 | 800 | 800 | 800 |
|---|---|---|---|---|
| Aging Temp, °C | 500 | 450 | 350 | 350 |
| Aging Time, hrs | 6 | 1.5 | 6 | 1.5 |
| Ultimate Tensile Strength, MPa | 775 | 810 | 872 | 882 |
| Yield Strength, MPa | 651 | 625 | 641 | 639 |
| Elongation, % | 23 | 20 | 18 | 17 |
| Reduction in Area, % | 74 | 70 | 60 | 59 |
| Young's Modulus, GPa | 86 | 84 | 88 | 87 |

The strength of these alloys is not very high, even after aging, since they had been air cooled (slow cooling as opposed to rapid cooling of water quenching) after forging.

Other Ti-13Nb-13Zr forgings, also of minimum 19mm diameter, were water quenched after forging, then heat treated. The mechanical properties of these forgings are recorded in Table VI.

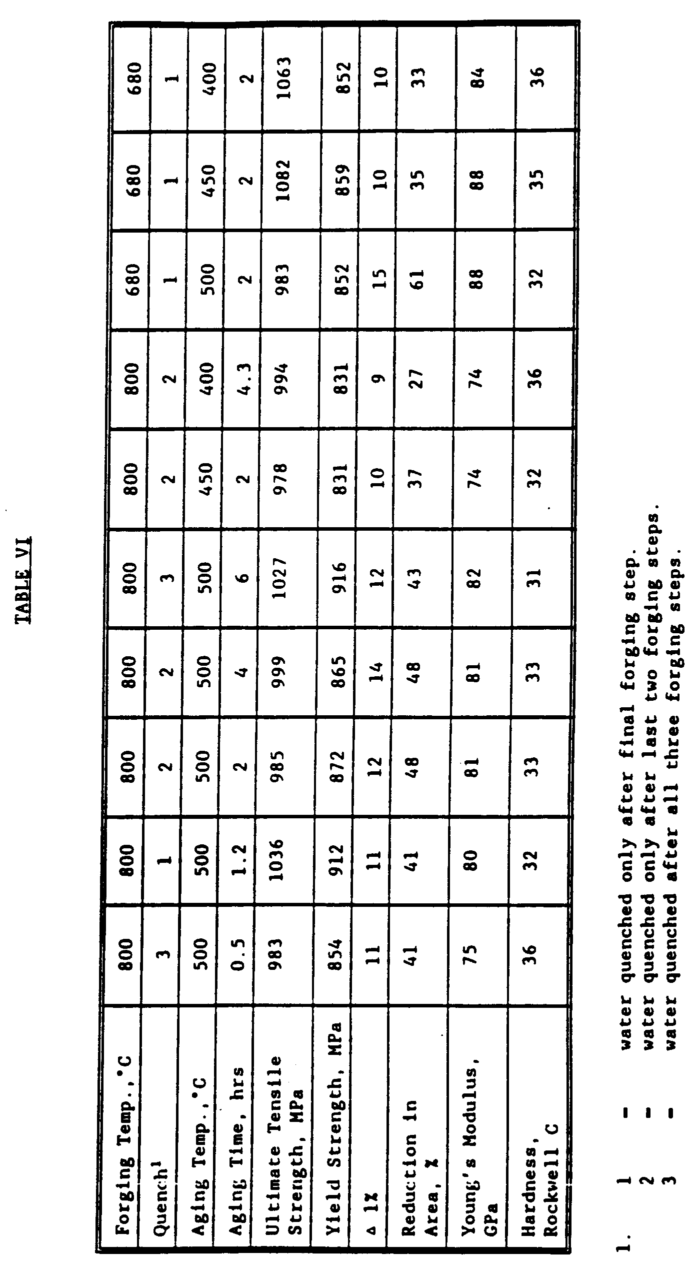

The alloy shows significant further improvement in strength while retaining a Young's modulus in the range 74-90 GPa. Hardness is also significantly greater than for the non-heat treated alloy as well as the reheated, quenched, and aged alloy of Example 2.

### Example 8

Several tests were performed to determine the effect on the physical properties of Ti-13Zr-13Nb when forged at different temperatures, aged at different temperatures for periods of time, and quenched under different conditions.

Water quenched 19mm minimum diameter samples from hip stems (proximal, mid, or distal sections) were heat treated at low temperatures for short times and their properties measured and recorded in Table VII.

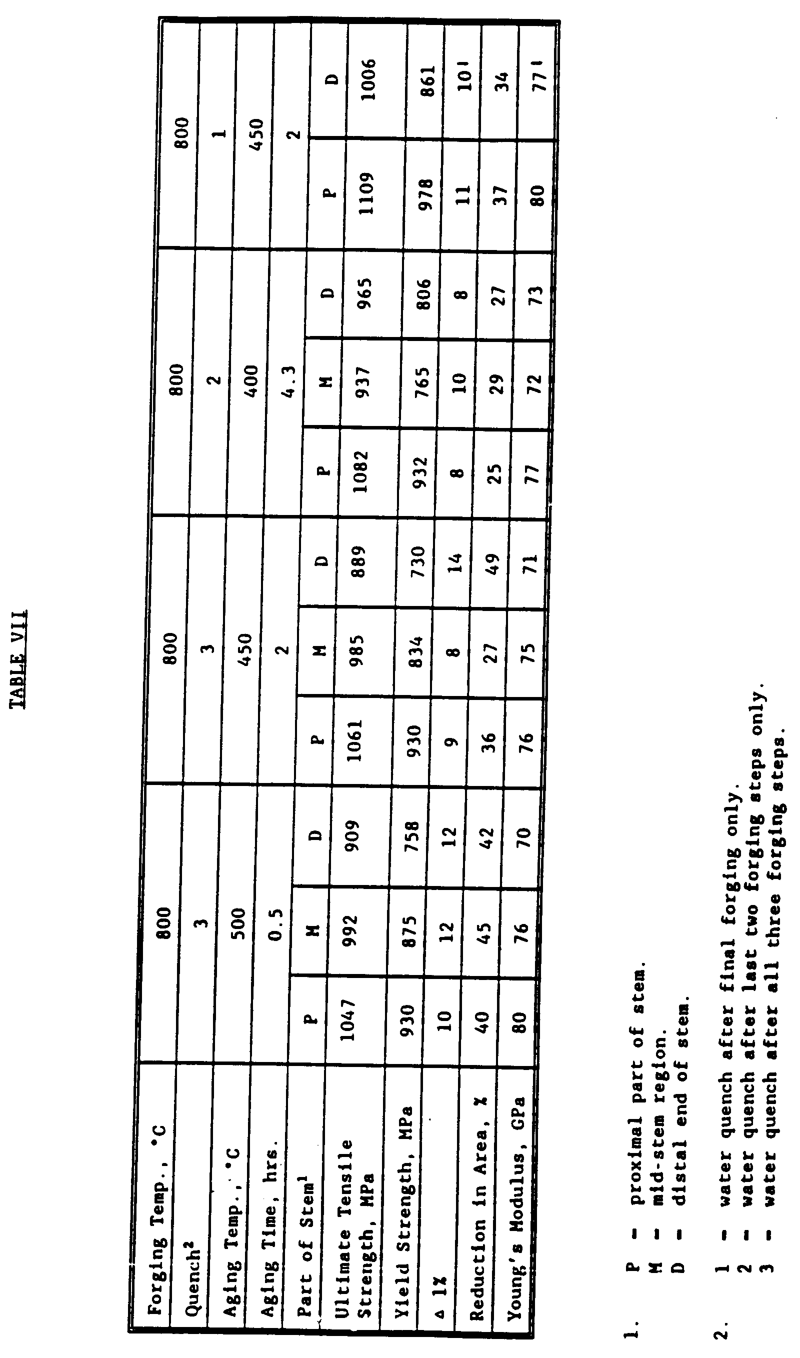

Forgings of 14mm minimum diameter were water quenched after the final forging step only, then aged at 500°C for 1, 2 or 3 hours. Their properties were:

**TABLE VIII**

| | | | |
|---|---|---|---|
| Forgings Temp, °C | 800 | 800 | 800 |
| Aging Temp, °C | 500 | 500 | 500 |
| Aging Time, hrs | 1 | 2 | 3 |
| Ultimate Tensile Strength, MPa | 953 | 960 | 1017 |
| Yield Strength, MPa | 802 | 811 | 898 |
| Elongation, % | 14 | 11 | 12 |
| Reduction in Area, % | 50 | 44 | 42 |
| Young's Modulus, GPa | 73 | 73 | 82 |

The forgings show an increase in both yield strength and tensile strength with time of aging.

### Example 9

A 12.5 inch long by 14 inch diameter segment was cut from an ingot of Ti-13Zr-13Nb which had been produced by arc melting. The plate was press forged to a 3 inch thickness at 1000°C. The press forged plate was then air annealed at 1100°C for 15-30 minutes before being hot rolled at 900°C to a 1.35 inch penultimate thickness. The hot rolled plate was then reheated to 900°C, hot rolled to 1.04 inch final thickness, and water quenched before being blasted and pickled. The mechanical properties of the plate were as follows:

**TABLE IXA**

| | Plate |
|---|---|
| Ultimate Tensile Strength (MPa) | 786±0 |
| Yield Strength (MPa) | 539±12 |
| Elongation (%) | 21±0 |
| Reduction in Area (%) | 51±5 |
| Young's Modulus (GPa) | 74±3 |
| Hardness, Rockwell C | 24.6±2.0 |

The hot worked and water quenched plate was then subjected to aging cycles consisting of a gradual heating up step and an isothermal aging step. The heating up appeared to produce a "preaging effect" on the material which enhanced subsequent aging response and produced a higher strength. The aging cycle included heating up the plate over a period of 1.5 to 5 hours up to 500°C (preaging), followed by 6 hours of aging at 500°C. The resultant mechanical properties of the plate are shown in Table IXB:

### TABLE IXB

From comparing Tables IXA and IXB, it is apparent that strength and hardness have increased significantly due to the aging process.

### Example 10

A 54 inch long by 14 inch diameter bar was cut from an ingot of Ti-13Zr-13Nb produced by arc melting. The bar was rotary forged to a 5.4 inch diameter at 875°C, then air annealed at 1050°C for 3 hours. Thereafter, the bar was rotary forged at 800°C to 2.5 inch diameter, and rotary forged at 750°C to 1.2 inch penultimate diameter. The swagged bar was then reheated to 925°C before being rotary swagged to a 1 inch final diameter. The bar was water quenched, blasted and pickled, and then centreless ground.

The resultant mechanical properties of this Ti-13Zr-13Nb bar were as follows:

**TABLE XA**

| | Bar |
|---|---|
| Ultimate Tensile Strength (MPa) | 722±9 |
| Yield Strength (MPa) | 463±10 |
| Elongation (%) | 26±0 |
| Reduction in Area (%) | 66±2 |
| Young's Modulus (GPa) | 79±9 |
| Hardness, Rockwell C | 24±2.7 |

The hot worked and water quenched bar was then subjected to aging cycles including a gradual heating up step and an isothermal aging step. Once again, the heating up appeared to produce a preaging effect on the bar, which enhanced subsequent aging response and produced a higher strength material. During aging, the bar was first gradually heated, over a period of 1.5 to about 5 hours, up to 500°C. This was followed by 6 hours of isothermal aging at 500°C. The resultant mechanical properties of the bar were as follows:

**TABLE XB**

| | |
|---|---|
| Ramp-up Time to 500°C (hr): | 2.5 |
| Ultimate Tensile Strength (MPa) | 1008±6 |
| Yield Strength (MPa) | 881±13 |
| Elongation (%) | 10±3 |
| Reduction in Area (A%) | 32±14 |
| Young's Modulus (GPa) | 82.4±2.3 |
| Hardness, Rockwell C | 31.5±0.6 |

As can be seen from a comparison of Table XA and Table XB, the strength and hardness of the bar increased significantly as a result of the aging and preaging processes.

The invention has been described with reference to its preferred embodiments. From this description, a person of ordinary skill in the art may appreciate changes that could be made in the invention which do not depart from the scope and spirit of the invention as described above and claimed hereafter.

## Claims

1. A biocompatible medical implant of low modulus and high strength for implantation into a living body where it is subject to corrosive effects of body fluids, said medical implant comprising:
a metallic alloy consisting essentially of:
(i) titanium;
(ii) from about 10 to 20 wt% niobium or from about 25 to about 50 wt% niobium; and
(iii) optionally up to about 20 wt% zirconium;
wherein said medical implant has an elastic modulus less than about 90 GPa and the corrosive effects of body fluids does not result in release of toxic or potentially toxic ions into the living body after surgical implantation of the implant into said body.

2. The medical implant according to claim 1 which is a cardiovascular implant.

3. The medical implant according to claims 1 or 2 consisting essentially of:
(i) titanium;
(ii) from about 10 to 20 wt% niobium; and
(iii) up to about 20 wt% zirconium.

4. The medical implant according to claims 1 or 2 consisting essentially of:
(i) titanium;
(ii) from about 35 to 50 wt% niobium; and
(iii) up to about 20 wt% zirconium.

5. The medical implant of claim 1 wherein the implant is selected from the group consisting of bone plates, bone screws, intramedullary rods, and compression hip screws.

6. The medical implant of claim 1 wherein the metallic alloy comprises tantalum in an amount sufficient to stabilise the β-phase without significantly affecting the modulus of elasticity of the implant.

7. The medical implant of claim 1 wherein the alloy is substantially in the β-phase and has a strength greater than about 620 MPa.

8. The medical implant of claim 1, wherein the alloy consists essentially of: 74 wt.% titanium, 13 wt.% niobium, and 13 wt.% zirconium.

9. The medical implant of claim 8 wherein the alloy is substantially in the β-phase and has a strength greater than about 620 MPa.

10. The medical implant of claim 1 further comprising at least a partial outer surface protective coating selected from the group consisting of the oxides, nitrides, carbides and carbonitrides of elements of the metal alloy.

11. The medical implant of claim 1 further comprising a protective coating of amorphous diamond-like carbon on at least a portion of an outer surface of the implant.

12. The medical implant of claim 1 wherein the metallic alloy is internally oxidised or nitrided beneath outer surfaces of the implant to produce a hardened medical implant.

13. The medical implant of claim 1 wherein the implant is selected from the components of a modular knee joint consisting of a femoral component and a tibial base component.

14. The medical implant of claim 13 wherein the metallic alloy is internally oxidised or nitrided beneath outer surfaces of the implant to produce a hardened medical implant.

15. The medical implant of claim 13 wherein the alloy is substantially in the β-phase and has a strength greater than about 620 MPa.

16. The medical implant of claim 13 wherein the alloy consists essentially of: 74 wt% titanium, 13 wt% niobium, and 13 wt% zirconium.

17. The medical implant of claim 13 further comprising a protective coating of amorphous diamond-like carbon on at least a portion of an outer surface of the implant.

18. The medical implant of claim 13 further comprising at least a partial outer surface protective coating selected from the group consisting of the oxides, nitrides, carbides and carbonitrides of elements of the metal alloy.

19. The medical implant according to claim 2 comprising a heart valve prosthesis for implantation in living body tissue of a patient, the heart valve having enhanced hemocompatibility, comprising:
(a) a valve body having an aperture through which blood is able to flow when the heart valve is implanted in a patient, the valve body fabricated from a metal alloy comprising:
(i) titanium;
(ii) from about 10 to about 20 wt% niobium or from about 35 to about 50 wt% niobium; and
(iii) optionally up to about 20 wt% zirconium;
(b) a flow control element able to move relative to the valve body to close the aperture in the valve body thereby blocking blood flow through the aperture; and
(c) means, attached to the valve body, for restraining said flow control component to close proximity to the aperture in the valve body.

20. The heart valve prosthesis of claim 19, wherein the metal alloy comprises from about 0.5 to about 20 wt.% zirconium.

21. The heart valve prosthesis of claim 20, wherein outer surfaces of the valve body are hardened by a process selected from the group consisting of oxygen diffusion hardening, nitrogen hardening, physical vapour deposition, and chemical vapour deposition.

22. The heart valve prosthesis of claim 19 further comprising a coating overlayed over outer surfaces comprising a medicament.

23. The heart valve prosthesis of claim 19 further comprising wear-resistant surfaces produced by a process selected from the group consisting of boronation and silver doping.

24. The medical implant according to claim 2 comprising a ventricular assist device including components with surfaces subject to mechanical wear and microfretting wear, the improvement comprising:
components fabricated from a metal alloy comprising:
(a) titanium;
(b) from about 10 to about 20 wt% niobium or from about 25 to 50 wt% niobium; and
(c) optionally up to about 20 wt% zirconium.

25. The ventricular assist device of claim 24, wherein the metal alloy comprises from about 0.5 to about 20 wt% zirconium.

26. The ventricular assist device of claim 25, wherein the surfaces subject to mechanical wear and microfretting wear are hardened by a process selected from the group consisting of oxygen diffusion hardening, nitrogen hardening, physical vapour deposition, and chemical vapour deposition.

27. The ventrical assist device of claim 24, wherein the surfaces are coated with a medicament.

28. The ventricular assist device of claim 24 further comprising a coating on the surfaces subject to mechanical and microfretting wear, the coating applied by a process selected from the group consisting of silver doping and boronation.

29. The medical implant according to claim 2 comprising a ventricular assist device including components with surfaces in contact with blood when the device is implanted in a patient, the improvement comprising:
said components fabricated from a metal alloy comprising:
(a) titanium;
(b) from about 10 to about 20 wt% niobium or from about 35 to about 50 wt% niobium; and
(c) optionally up to about 20 wt% zirconium.

30. The device of claim 29, wherein the metal alloy comprises from about 0.5 to about 20 wt% zirconium.

31. The device of claim 30, wherein the surfaces in contact with blood are hardened by a process selected from the group consisting of oxygen diffusion hardening, nitrogen hardening, physical vapour deposition, and chemical vapour deposition.

32. The device of claim 29, wherein the surfaces in contact with blood are coated with a composition comprising a medicament.

33. The medical implant according to claim 2 comprising a total artificial heart device for implantation into a chest cavity of a patient, the device including components with surfaces subject to mechanical wear and microfretting wear when in use in the patient, the improvement comprising:
components fabricated from a metal alloy comprising:
(a) titanium;
(b) from about 10 to about 20 wt% niobium or from about 35 to about 50 wt% niobium; and
(c) optionally up to about 20 wt% zirconium.

34. The device of claim 33, wherein the metal alloy comprises from about 0.5 to about 20 wt% zirconium.

35. The device of claim 34, wherein the surfaces subject to mechanical wear and microfretting wear are hardened by a process selected from the group consisting of oxygen diffusion hardening, nitrogen hardening, physical vapour deposition, and chemical vapour deposition.

36. The device of claim 33, wherein the surfaces subject to mechanical wear and microfretting wear are hardened by coating with a process selected from the group consisting of silver doping and boronation.

37. The medical implant according to claim 2 comprising a total artificial heart device for implantation into a chest cavity of a patient, the device including components presenting surfaces that are in contact with blood when in use in the patient, the improvement wherein the components are fabricated from an alloy comprising:
(a) titanium
(b) from about 10 to about 20 wt% niobium or from about 35 to about 50 wt% niobium; and
(c) optionally up to about 20 wt% zirconium.

38. The device of claim 37, wherein the metal alloy comprises from about 0.5 to about 20 wt% zirconium.

39. The device of claim 38, wherein outer surfaces of the components are hardened by a process selected from the group consisting of oxygen diffusion hardening, nitrogen hardening, physical vapour deposition, and chemical vapour deposition.

40. The device of claim 37, wherein the surfaces in contact with blood are coated overlayed over outer surfaces comprising medicament.

41. The medical implant according to claim 2 comprising a flexible guide wire for insertion into a living body to perform surgical operations, the guide wire comprising:
(a) an elongate, flexible guide wire body having a distal end, for insertion into a patient and into a catheter, and a proximal end for controlling the guide wire;
(b) an elongate guide wire core disposed internally along the longitudinal axis of the elongate body, said core comprising a metal alloy comprising:
(i) titanium;
(ii) from about 10 to about 20 wt% niobium or from about 35 to 50 wt% niobium; and
(iii) optionally up to about 20 wt% zirconium.

42. The guide wire of claim 41 further comprising a hardened cutting edge on the distal end of the elongate body.

43. The wire of claim 41, wherein the metal alloy comprises from about 0.5 to about 20 wt% zirconium.

44. The wire of claim 43, wherein the core has a hardened outer surface produced by a process selected from the group consisting of oxygen diffusion hardening, nitrogen hardening, physical vapour deposition, and chemical vapour deposition.

45. The wire of claim 41, where surfaces of the wire that come into contact with body tissue are coated with a composition comprising a medicament.

46. The wire of claim 41, further comprising a hardened outer surface, said hardened outer surface produced by a process selected from the group consisting of silver doping and boronation.

47. The medical implant according to claim 2 comprising an expandable stent for supporting a blood, urinary, or gastrointestinal vessel from collapsing inward, the stent comprising:
(a) a radially outwardly expandable substantially cylindrical stent body of a metal alloy comprising:
(i) titanium;
(ii) from about 10 to about 20 wt% niobium or from about 35 to about 50 wt% niobium; and
(iii) optionally up to about 20 wt% zirconium;
the stent body in its unexpected state sized for ease of insertion into a vessel and in expanded state sized for propping open a vessel, the stent body having a bore therethrough for receiving a means for expanding the body radially outwardly.

48. The stent of claim 47, wherein the metal alloy comprises from about 0.5 to about 20 wt% zirconium.

49. The stent of claim 48, wherein surfaces of the stent are hardened by a process selected from the group consisting of oxygen diffusion hardening, nitrogen hardening, and physical vapour deposition, and chemical vapour deposition.

50. The stent of claim 47, wherein surfaces of the stent are coated with a composition comprising a medicament.

51. The stent of claim 47 further comprising a coating on surfaces of the stent, said coating applied by a process selected from the group consisting of silver doping and boronation.

52. The medical implant according to claim 2 comprising a biocompatible lead or sensor for conducting electrical signals to or from an organ in a living body, the lead comprising:
an elongate flexible body having distal and proximal ends, the flexible body comprising:
(a) an electrically conductive core of a metal alloy comprising:
(i) titanium
(ii) from about 10 to about 20 wt% niobium or from about 35 to about 50 wt% niobium; and
(iii) optionally up to about 20 wt% zirconium; for carrying the electrical signals.

53. The lead of claim 52, wherein the metal alloy comprises from about 0.5 to about 20 wt% zirconium.

54. The lead of claim 53 further comprising a thin non-electrically conductive layer surrounding the electrically conductive core.

55. The lead of claim 53 further comprising a coating on the non-electrically conducting layer, said coating comprising a medicament.

56. The medical implant according to claim 2 comprising a low modulus, biocompatible, percutaneous implant that penetrates the skin of a living body and thereby protrudes from the body, the implant comprising:
(a) a low modulus metallic implant body fabricated from a metal alloy comprising:
(i) titanium;
(ii) from about 10 to about 20 wt% niobium or from about 35 to about 50 wt% niobium; and
(iii) optionally up to about 20 wt% zirconium;
said implant body having a first end for insertion into said patient and a portion with a second end for extending outside of said patient.

57. The percutaneous implant of claim 56, wherein the metal alloy comprises from about 0.5 to about 20 wt% zirconium.

58. The percutaneous implant of claim 57 further comprising a hardened outer surface, said hardened outer surface produced by a process selected from the group consisting of oxygen diffusion hardening, nitrogen hardening, physical vapour deposition, and chemical vapour deposition.

59. The percutaneous implant of claim 56, further comprising a coating on surfaces of the implant body, said coating comprising a medicament.

60. The percutaneous implant of claim 56 further comprising a wear-resistant layer on surfaces of the implant body, the wear-resistant layer produced by a process selected from the group consisting of silver doping and boronation.

61. The medical implant according to claim 2 comprising an external mechanical heart including Components with surfaces subject to mechanical wear and microfretting wear, the improvement comprising:
components of a metal alloy comprising:
(a) titanium;
(b) from about 10 to about 20 wt% niobium or from about 35 to about 50 wt% niobium; and
(c) optionally up to about 20 wt% zirconium.

62. The mechanical heart of claim 61, wherein the metal metal alloy comprises from about 0.5 to about 20 wt% zirconium.

63. The mechanical heart of claim 62 further comprising a hardened outer surface on the components subject to mechanical and microfretting wear.

64. The mechanical heart of claim 61 further comprising a wear-resistant coating on surfaces subject to mechanical wear and microfretting wear, the wear-resistant coating produced by a process selected from the group consisting of silver doping and boronation.

65. The medical implant according to claim 2 comprising an external mechanical heart including components that contact blood when said heart is used to supply blood to a patient, the improvement comprising the components fabricated from a metal alloy comprising:
(a) titanium;
(b) from about 10 to about 20 wt% niobium or from about 35 to about 50 wt% niobium; and
(c) optionally up to about 20 wt% zirconium.

66. The mechanical heart of claim 65, wherein the metal alloy comprises from about 0.5 to about 20 wt% zirconium.

67. The mechanical heart of claim 65 further comprising a coating on outer surfaces of the components that contact blood, the coating comprising a medicament.

68. The medical implant according to claim 2 comprising a vascular graft of enhanced durability, crush-resistance, low thrombogenicity, and hemocompatibility, said graft comprising:
(a) titanium;
(b) from about 10 to about 20 wt% niobium or from about 35 to about 50 wt% niobium; and
(c) optionally up to about 20 wt% zirconium;
said tubular body having a bore therethrough, said tubular body sized to replace a section of removed blood vessel.

69. The vascular graft of claim 68, wherein the metal alloy comprises from about 0.5 to about 20 wt.% zirconium.

70. The vascular graft of claim 69 further comprising a hardened surface on the tubular body, said hardened surface produced by a process selected from the group consisting of oxygen diffusion hardening, nitrogen hardening, physical vapour deposition and chemical vapour deposition.

71. The vascular graft of claim 68 further comprising a coating on the tubular body, said coating selected from the group consisting a heparin, phosphatidyl choline, and a medicament.

72. The vascular graft of claim 68, further comprising a wear-resistant coating on the tubular body, said wear-resistant coating applied by a process selected from the group consisting of silver doping and boronation.
